# EUROPEAN PATENT SPECIFICATION

(11) **EP 3 363 379 B1**
(45) Date of publication and mention of the grant of the patent: **13.05.2020**
(21) Application number: 18157178.7
(22) Date of filing: 16.02.2018
(51) Int. Cl.: A61B 17/072

(54) **END EFFECTOR WITH ADJUNCT MATERIALS**
ENDEFFEKTOR MIT ERGÄNZUNGSMATERIALIEN
EFFECTEUR D'EXTRÉMITÉ AVEC MATIÈRES AUXILIAIRES

(30) Priority: 17.02.2017 US 201715436384
(43) Date of publication of application: 22.08.2018
(73) Proprietor: Ethicon LLC, 00969 Guaynabo (PR)
(72) Inventor: SHELTON, IV Frederick E., Cincinnati, Ohio 45242 (US); HARRIS, Jason L., Cincinnati, Ohio 45242 (US); VENDELY, Michael J., Cincinnati, Ohio 45242 (US)
(74) Representative: Carpmaels & Ransford LLP

(56) References cited:
- EP-A2- 2 311 386
- AU-A1- 2012 250 107
- US-A1- 2010 249 805
- US-A1- 2013 256 377
- US-A1- 2014 131 418

## Description

### FIELD

The present disclosure relates generally to adjunct materials used in conjunction with an end effector of a surgical instrument.

### BACKGROUND

Surgical staplers are used in surgical procedures to close openings in tissue, blood vessels, ducts, shunts, or other objects or body parts involved in the particular procedure. The openings can be naturally occurring, such as passageways in blood vessels or an internal organ like the stomach, or they can be formed by the surgeon during a surgical procedure, such as by puncturing tissue or blood vessels to form a bypass or an anastomosis, or by cutting tissue during a stapling procedure.

Most staplers have a handle with an elongate shaft having a pair of movable opposed jaws formed on an end thereof for holding and forming staples therebetween. The staples are typically contained in a staple cartridge, which can house multiple rows of staples and is often disposed in one of the two jaws for ejection of the staples to the surgical site. In use, the jaws are positioned so that the object to be stapled is disposed between the jaws, and staples are ejected and formed when the jaws are closed and the device is actuated. Some staplers include a knife configured to travel between rows of staples in the staple cartridge to longitudinally cut and/or open the stapled tissue between the stapled rows.

While surgical staplers have improved over the years, a number of problems still present themselves. One common problem is that leaks can occur due to the staple forming holes when penetrating the tissue or other object in which it is disposed. Blood, air, gastrointestinal fluids, and other fluids can seep through the openings formed by the staples, even after the staple is fully formed. The tissue being treated can also become inflamed due to the trauma that results from stapling. Still further, staples, as well as other objects and materials that can be implanted in conjunction with procedures like stapling, generally lack some characteristics of the tissue in which they are implanted. For example, staples and other objects and materials can lack the natural flexibility of the tissue in which they are implanted. A person skilled in the art will recognize that it is often desirable for tissue to maintain as much of its natural characteristics as possible after staples are disposed therein.

Accordingly, there remains a need for improved devices and methods for stapling tissue, blood vessels, ducts, shunts, or other objects or body parts such that leaking and inflammation is minimized while substantially maintaining the natural characteristics of the treatment region.

US 2010/02495 A1 describes a surgical stapling instrument, which has retention member movably mounted on upper side of member, where end of retention component is releasably secured between distal face of retention member and proximal face of member.

US 2013/02141 A1 describes an anvil-attachable layer and a retainer for aligning and attaching the layer to an anvil of a surgical stapler. Embodiments of the anvil-attachable layer can include one or more attachment features extending from the layer.

EP 2 311 386 A2 describes a surgical stapling apparatus including a cartridge assembly defining a first tissue contacting surface, an anvil assembly defining a second tissue contacting surface, and a surgical buttress releasably secured to at least one of the first tissue contacting surface and the second tissue contacting surface.

### SUMMARY

In some aspects, an end effector for a surgical instrument is provided that in some embodiments includes a fist jaw, a second jaw opposing the first jaw, an attachment feature, and an adjunct material. The first jaw has a cartridge with a plurality of staple cavities configured to seat staples therein, the staple cavities opening on a tissue-facing surface of the cartridge. The second jaw has an anvil with a plurality of staple forming cavities formed on a tissue-facing surface thereof. The first and second jaws are configured to clamp tissue therebetween. The attachment feature includes a retaining filament having an intermediate portion and first and second ends disposed on opposed sides of the intermediate portion, each of the first and second ends having an end feature configured to mate with at least one jaw of the first and second jaws. The adjunct material releasably retained on the at least one jaw by the attachment feature is arranged such that at least a part of the intermediate portion is disposed over the adjunct material and such that the first and second ends are spaced apart.

Furthermore, the end effector includes at least one first retaining member disposed at one side of a tissue-facing surface of the at least one jaw in proximity to one edge of the tissue-facing surface of the at least one jaw, and at least one second retaining member disposed at another, opposed side of the tissue-facing surface of the at least one jaw in proximity to another, opposed edge of the tissue-facing surface. The first and second retaining members are configured to engage the part of the intermediate portion disposed over the adjunct material. Additionally, at least one of the first and second retaining members is or includes a pair of adjacent posts, each configured to engage at least a part of the intermediate portion of the retaining filament.

The adjunct material can include at least one cut-out formed therein, and the adjunct material can be releasably retained on the at least one jaw such that at least one of the first and second retaining members is disposed within the cut-out.

The first and second end features can be disposed on a side of the at least one jaw that is opposed to a tissue-facing surface thereof, such that the first and second ends are spaced apart across a cutting element channel of the at least one jaw extending longitudinally across a mid-portion of the jaw. In some embodiments, at least the intermediate portion of the retaining filament is coupled to the at least one jaw using an adhesive material.

In some embodiments, the end effector includes at least one roughened portion on a side of the at least one jaw that is opposed to a tissue-facing surface thereof, wherein the intermediate portion of the retaining filament encompasses opposed sides walls of the at least one jaw, and wherein the first and second ends comprise leaf members configured to frictionally engage a corresponding roughened portion of the at least one roughened portion. In some embodiments, the end effector further includes at least one second roughened portion on at least one of the opposed side walls, wherein portions of the intermediate portion of the retaining filament that encompass the opposed side walls frictionally engage a corresponding second roughened portion on one of the side walls.

In some implementations, the end effector includes at least one spindle-type retaining member formed on a side of the at least one jaw that is opposed to a tissue-facing surface thereof. In such implementations, at least one of the first and second ends of the retaining filament is configured to be retained via the spindle-type retaining member. The spindle-type retaining member can vary in many different ways. For example, it can have at least one recess formed thereon and configured to retain therein a portion of the at least one of the first and second ends.

In some implementations, the end effector further has a first pair of recesses formed in a tissue-facing surface of the at least one jaw, the first pair of recesses being spaced from opposed edges of the tissue-facing surface, and a second pair of recesses formed in the adjunct material at locations thereof corresponding to the first pair of recesses. Parts of the intermediate portion of the retaining filament can be configured to extend through the second pair of recesses so that the first and second ends are allowed within the first pair of recesses. The first and second ends of the retaining filament can include or can be deformable elements configured to be reversibly deformed when allowed within the first pair of recesses. In at least one embodiment, the deformable elements are or include t-shaped barb members. In some embodiments, the at least one jaw is the first jaw and the first pair of recesses is formed at a distal end of the jaw outside an outer perimeter of the plurality of staple cavities.

In other aspects, not forming part of the invention, an end effector for a surgical instrument is provided that in some embodiments includes a first jaw, a second jaw opposing the first jaw, a first adjunct material configured to be releasably retained on the first jaw, a second adjunct material configured to be releasably retained on the second jaw, and an attachment feature. The first jaw has a cartridge with a plurality of staple cavities configured to seat staples therein, the staple cavities opening on a tissue-facing surface of the cartridge. The second jaw has an anvil with a plurality of staple forming cavities formed on a tissue-facing surface thereof. The first and second jaws are configured to clamp tissue therebetween. The first adjunct material has proximal and distal ends and a first mating feature at the proximal end thereof. The second adjunct material, having proximal and distal ends, is separate from the first adjunct material and has a second mating feature at the proximal end thereof. The attachment feature is formed at a proximal end of the end effector and is configured to mate with at least one of the first and second mating features of the adjunct materials.

The end effector can vary in many different ways. For example, the first and second mating features can be or can include open-end loop features configured to movably encompass the attachment feature. The first and second mating features can be configured to engage the attachment feature such that the first mating features engage the attachment feature at locations different from locations at which the second mating features engage the attachment feature.

In yet other aspects, not forming part of the invention, an end effector for a surgical instrument is provided that in some embodiments includes a first jaw, a second jaw opposing the first jaw, a first adjunct material configured to be releasably retained on the first jaw, and a second adjunct material configured to be releasably retained on the second jaw. The first jaw has a cartridge with a plurality of staple cavities configured to seat staples therein, the staple cavities opening on a tissue-facing surface

of the cartridge. The second jaw has an anvil with a plurality of staple forming cavities formed on a tissue-facing surface thereof. The first and second jaws are configured to clamp tissue therebetween. The first adjunct material has proximal and distal ends and a first mating feature at the proximal end thereof. The second adjunct material, having proximal and distal ends, is separate from the first adjunct material and has a second mating feature at the proximal end thereof. The first and second mating features of the first and second adjunct materials are configured to couple to one another to thereby couple the first and second adjuncts to one another.

The end effector can vary in many different ways. For example, in some implementations, the attachment feature is or includes a slot formed at the proximal end of the first adjunct material, and the second mating feature is or includes a tab extending from the proximal end of the second adjunct material and configured to be received within the slot.

### BRIEF DESCRIPTION OF DRAWINGS

This disclosure will be more fully understood from the following detailed description taken in conjunction with the accompanying drawings, in which:
FIG. 1 is a perspective view of one embodiment of a surgical stapler;
FIG. 2 is an exploded view of a distal portion of the surgical stapler of FIG. 1;
FIG. 3 is a perspective view of a firing bar of the surgical stapler of FIG. 1;
FIG. 4 is a perspective view of another embodiment of a surgical stapler;
FIG. 5 is a perspective view of yet another embodiment of a surgical stapler;
FIG. 6 is a perspective view of a jaw of an end effector having an adjunct material releasably mounted thereon using an attachment feature in accordance with the described techniques;
FIG. 7 is a perspective partial view of the jaw with the adjunct material of FIG. 6;
FIG. 8 is a perspective view of a side of the jaw of FIG. 6 that is opposed to a tissue-contacting side thereof, illustrating end features of the attachment feature;
FIG. 9 is another perspective view of a jaw of an end effector having an adjunct material releasably mounted thereon using an attachment feature in accordance with the described techniques;
FIG. 10 is a schematic diagram illustrating an example of a roughness portion that can be formed on the jaw of FIG. 6 and the jaw of FIG. 9;
FIG. 11 is a perspective view of an upper side of a jaw of an end effector having an adjunct material releasably mounted thereon using a spindle-type attachment feature in accordance
   with the described techniques;
   The attachment features shown in figures 12-19B do not form part of the invention.
FIG. 12 is a perspective view of a jaw of an end effector configured to releasably retain thereon an adjunct material using an attachment feature in accordance with the described techniques;
FIG. 13 is a perspective, partially transparent view of the jaw of FIG. 12, illustrating the attachment feature releasably retaining the adjunct material on the jaw;
FIG. 14 is a perspective view of a jaw of an end effector configured to releasably retain thereon first and second adjunct materials;
FIG. 15 is a perspective view of the jaw of FIG. 14, illustrating the first and second adjunct materials releasably retained on the jaw;
FIG. 16 is another perspective view of a jaw of an end effector configured to releasably retain thereon first and second adjunct materials;
FIG. 17 is a perspective view of a portion of the first and second adjunct materials of FIG. 16, illustrating a tab in one of the adjunct materials engaging with a slot in another one of the adjunct materials;
FIG. 18 is a perspective view of a jaw of an end effector configured to releasably retain thereon an adjunct material;
FIG. 19A is a schematic diagram illustrating a portion of the adjunct material of FIG. 18; and
FIG. 19B is a schematic diagram illustrating the portion of the adjunct material of FIG. 19A in engagement with a portion of the jaw of FIG. 18.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

It will be appreciated that the terms "proximal" and "distal" are used herein with reference to a user, such as a clinician, gripping a handle of an instrument. Other spatial terms such as "front" and "back" similarly correspond respectively to distal and proximal. It will be further appreciated that for convenience and clarity, spatial terms such as "vertical" and "horizontal" are used herein with respect to the drawings. However, surgical instruments are used in many orientations and positions, and these spatial terms are not intended to be limiting and absolute.

In some embodiments, the devices and methods described herein are provided for open surgical procedures, and in other embodiments, the devices and methods are provided for laparoscopic, endoscopic, and other minimally invasive surgical procedures. The devices may be fired directly by a human user or remotely under the direct control of a robot or similar manipulation tool. However, a person skilled in the art will appreciate that the various methods and devices disclosed herein can be used in numerous surgical procedures and applications. Those skilled in the art will further appreciate that the various instruments disclosed herein can be inserted into a body in any way, such as through a natural orifice, through an incision or puncture hole formed in tissue, or through an access device, such as a trocar cannula. For example, the working portions or end effector portions of the instruments can be inserted directly into a patient's body or can be inserted through an access device that has a working channel through which the end effector and elongated shaft of a surgical instrument can be advanced.

It can be desirable to use one or more biologic materials and/or synthetic materials, collectively referred to herein as "adjuncts," in conjunction with surgical instruments to help improve surgical procedures. While a variety of different surgical end effectors can benefit from the use of adjuncts, in some exemplary embodiments the end effector can be a surgical stapler. When used in conjunction with a surgical stapler, the adjunct(s) can be disposed between and/or on jaws of the stapler, incorporated into a staple cartridge disposed in the jaws, or otherwise placed in proximity to the staples. When staples are deployed, the adjunct(s) can remain at the treatment site with the staples, in turn providing a number of benefits. For example, the adjunct(s) may reinforce tissue at the treatment site, preventing tearing or ripping by the staples at the treatment site. Tissue reinforcement may be needed to keep the staples from tearing through the tissue if the tissue is diseased, is healing from another treatment such as irradiation, medications such as chemotherapy, or other tissue property altering situation. In some instances, the adjunct(s) may minimize tissue movement in and around the staple puncture sites that can occur from tissue deformation that occurs after stapling (e.g., lung inflation, gastrointestinal tract distension, etc.). It will be recognized by one skilled in the art that a staple puncture site may serve as a stress concentration and that the size of the hole created by the staple will grow when the tissue around it is placed under tension. Restricting the tissues movement around these puncture sites can minimize the size the holes may grow to under tension. In some instances, the adjunct(s) can be configured to wick or absorb beneficial fluids, e.g., sealants, blood, glues, that further promote healing, and in some instances, the adjunct(s) can be configured to degrade to form a gel, e.g., a sealant, that further promotes healing. In some instances, the adjunct(s) can be used to help seal holes formed by staples as they are implanted into tissue, blood vessels, and various other objects or body parts. The adjunct(s) may also affect tissue growth through the spacing, positioning and/or orientation of any fibers or strands associated with the adjunct(s).

Furthermore, in some circumstances, an adjunct can be useful in distributing pressure applied by the staple thereby reducing the possibility of a staple pulling through a tissue (which can be friable) and failing to fasten the tissue as intended (so-called "cheese wiring"). Additionally, the adjunct can be at least partially stretchable and can thus allow at least partial natural motion of the tissue (e.g., expansion and contraction of lung tissue during breathing). In some embodiments, a staple line can be flexible as described, for example, in U.S. Pat. Pub. No. 2016/0089142 entitled "Method for Creating a Flexible Staple Line," filed on September 26, 2014.

### SURGICAL STAPLING INSTRUMENTS

A variety of surgical instruments can be used in conjunction with the adjunct(s) and/or medicant(s) disclosed herein. "Adjuncts" are also referred to herein as "adjunct materials." The surgical instruments can include surgical staplers. A variety of surgical staplers can be used, for example, linear surgical staplers and circular staplers. In general, a linear stapler can be configured to create longitudinal staple lines and can include elongate jaws with a cartridge coupled thereto containing longitudinal staple rows. The elongate jaws can include a knife or other cutting element capable of creating a cut between the staple rows along tissue held within the jaws. In general, a circular stapler can be configured to create annular staple lines and can include circular jaws with a cartridge containing annular staple rows. The circular jaws can include a knife or other cutting element capable of creating a cut inside of the rows of staples to define an opening through tissue held within the jaws. The staplers can be used on a variety of tissues in a variety of different surgical procedures, for example in thoracic surgery or in gastric surgery.

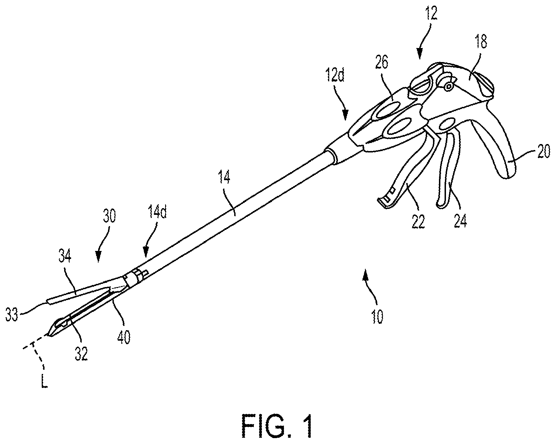

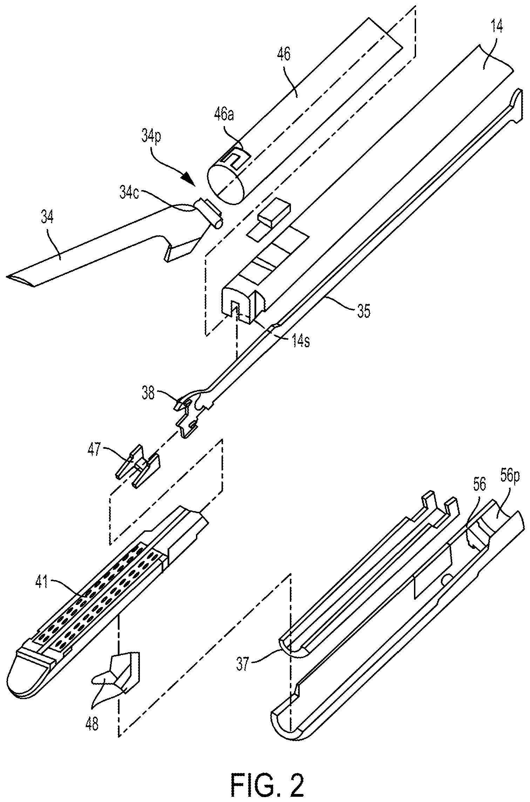

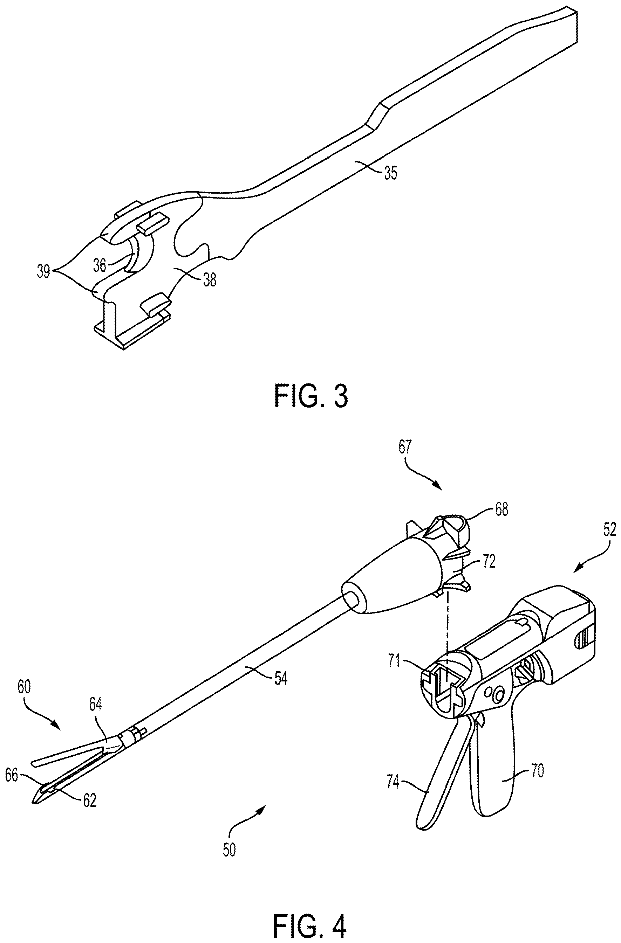

FIG. 1 illustrates one example of a linear surgical stapler 10 suitable for use with one or more adjunct(s) and/or medicant(s). The stapler 10 generally includes a handle assembly 12, a shaft 14 extending distally from a distal end 12d of the handle assembly 12, and an end effector 30 at a distal end 14d of the shaft 14. The end effector 30 has opposed lower and upper jaws 32, 34, although other types of end effectors can be used with the shaft 14, handle assembly 12, and components associated with the same. As shown in FIG. 2, the lower jaw 32 has a staple channel 56 (see FIG. 2) configured to support a staple cartridge 40, and the upper jaw 34 has an anvil surface 33 that faces the lower jaw 32 and that is configured to operate as an anvil to help deploy staples of the staple cartridge 40 (the staples are obscured in FIGS. 1 and 2). At least one of the opposed lower and upper jaws 32, 34 is moveable relative to the other lower and upper jaws 32, 34 to clamp tissue and/or other objects disposed therebetween. In some implementations, one of the opposed lower and upper jaws 32, 34 may be fixed or otherwise immovable. In some implementations, both of the opposed lower and upper jaws 32, 34 may be movable. Components of a firing system can be configured to pass through at least a portion of the end effector 30 to eject the staples into the clamped tissue. In various implementations a knife blade 36 (see FIG. 3) or other cutting element can be associated with the firing system to cut tissue during the stapling procedure. The cutting element can be configured to cut tissue at least partially simultaneously with the staples being ejected. In some circumstances, it may be advantageous if the tissue is cut after the staples have been ejected and the tissue is secured. Thus, if a surgical procedure requires that a tissue captured between the jaws be severed, the knife blade 36 is advanced to sever the tissue grasped between the jaws after the staples have been ejected from the staple cartridge 40.

Operation of the end effector 30 can begin with input from a user, e.g., a clinician, a surgeon, etc., at the handle assembly 12. The handle assembly 12 can have many different configurations designed to manipulate and operate the end effector 30 associated therewith. In the illustrated example, the handle assembly 12 has a pistol-grip type housing 18 with a variety of mechanical and/or electrical components disposed therein to operate various features of the instrument 10. For example, the handle assembly 12 can include a rotation knob 26 mounted adjacent the distal end 12d thereof which can facilitate rotation of the shaft 14 and/or the end effector 30 with respect to the handle assembly 12 about a longitudinal axis L of the shaft 14. The handle assembly 12 can further include clamping components as part of a clamping system actuated by a clamping trigger 22 and firing components as part of the firing system that are actuated by a firing trigger 24. The clamping and firing triggers 22, 24 can be biased to an open position with respect to a stationary handle 20, for instance by a torsion spring. Movement of the clamping trigger 22 toward the stationary handle 20 can actuate the clamping system, described below, which can cause the jaws 32, 34 to collapse towards each other and to thereby clamp tissue therebetween. Movement of the firing trigger 24 can actuate the firing system, described below, which can cause the ejection of staples from the staple cartridge 40 disposed therein and/or the advancement the knife blade 36 to sever tissue captured between the jaws 32, 34. A person skilled in the art will recognize that various configurations of components for a firing system, mechanical, hydraulic, pneumatic, electromechanical, robotic, or otherwise, can be used to eject staples and/or cut tissue.

As shown in FIG. 2, the end effector 30 of the illustrated implementation has the lower jaw 32 that serves as a cartridge assembly or carrier and the opposed upper jaw 34 that serves as an anvil. The staple cartridge 40, having a plurality of staples therein, is supported in a staple tray 37, which in turn is supported within a cartridge channel of the lower jaw 32. The upper jaw 34 has a plurality of staple forming pockets (not shown), each of which is positioned above a corresponding staple from the plurality of staples contained within the staple cartridge 40. The upper jaw 34 can be connected to the lower jaw 32 in a variety of ways, although in the illustrated implementation the upper jaw 34 has a proximal pivoting end 34p that is pivotally received within a proximal end 56p of the staple channel 56, just distal to its engagement to the shaft 14. When the upper jaw 34 is pivoted downwardly, the upper jaw 34 moves the anvil surface 33 and the staple forming pockets formed thereon move toward the opposing staple cartridge 40.

Various clamping components can be used to effect opening and closing of the jaws 32, 34 to selectively clamp tissue therebetween. As illustrated, the pivoting end 34p of the upper jaw 34 includes a closure feature 34c distal to its pivotal attachment with the staple channel 56. Thus, a closure tube 46, whose distal end includes a horseshoe aperture 46a that engages the closure feature 34c, selectively imparts an opening motion to the upper jaw 34 during proximal longitudinal motion and a closing motion to the upper jaw 34 during distal longitudinal motion of the closure tube 46 in response to the clamping trigger 22. As mentioned above, in various implementations, the opening and closure of the end effector 30 may be effected by relative motion of the lower jaw 32 with respect to the upper jaw 34, relative motion of the upper jaw 34 with respect to the lower jaw 32, or by motion of both jaws 32, 34 with respect to one another.

The firing components of the illustrated implementation includes a firing bar 35, as shown in FIG. 3, having an E-beam 38 on a distal end thereof. The firing bar 35 is encompassed within the shaft 14, for example in a longitudinal firing bar slot 14s of the shaft 14, and guided by a firing motion from the handle 12. Actuation of the firing trigger 24 can affect distal motion of the E-beam 38 through at least a portion of the end effector 30 to thereby cause the firing of staples contained within the staple cartridge 40. As illustrated, guides 39 projecting from a distal end of the E-Beam 38 can engage a wedge sled 47, shown in FIG. 2, which in turn can push staple drivers 48 upwardly through staple cavities 41 formed in the staple cartridge 40. Upward movement of the staple drivers 48 applies an upward force on each of the plurality of staples within the cartridge 40 to thereby push the staples upwardly against the anvil surface 33 of the upper jaw 34 and create formed staples.

In addition to causing the firing of staples, the E-beam 38 can be configured to facilitate closure of the jaws 32, 34, spacing of the upper jaw 34 from the staple cartridge 40, and/or severing of tissue captured between the jaws 32, 34. In particular, a pair of top pins and a pair of bottom pins can engage one or both of the upper and lower jaws 32, 34 to compress the jaws 32, 34 toward one another as the firing bar 35 advances through the end effector 30. Simultaneously, the knife 36 extending between the top and bottom pins can be configured to sever tissue captured between the jaws 32, 34.

In use, the surgical stapler 10 can be disposed in a cannula or port and disposed at a surgical site. A tissue to be cut and stapled can be placed between the jaws 32, 34 of the surgical stapler 10. Features of the stapler 10 can be maneuvered as desired by the user to achieve a desired location of the jaws 32, 34 at the surgical site and the tissue with respect to the jaws 32, 34. After appropriate positioning has been achieved, the clamping trigger 22 can be pulled toward the stationary handle 20 to actuate the clamping system. The clamping trigger 22 can cause components of the clamping system to operate such that the closure tube 46 advances distally through at least a portion of the shaft 14 to cause at least one of the jaws 32, 34 to collapse towards the other to clamp the tissue disposed therebetween. Thereafter, the firing trigger 24 can be pulled toward the stationary handle 20 to cause components of the firing system to operate such that the firing bar 35 and/or the E-beam 38 are advanced distally through at least a portion of the end effector 30 to effect the firing of staples and optionally to sever the tissue captured between the jaws 32, 34.

Another example of a surgical instrument in the form of a linear surgical stapler 50 is illustrated in FIG. 4. The stapler 50 can generally be configured and used similar to the stapler 10 of FIG. 1. Similar to the surgical instrument 10 of FIG. 1, the surgical instrument 50 includes a handle assembly 52 with a shaft 54 extending distally therefrom and having an end effector 60 on a distal end thereof for treating tissue. Upper and lower jaws 64, 62 of the end effector 60 can be configured to capture tissue therebetween, staple the tissue by firing of staples from a cartridge 66 disposed in the lower jaw 62, and/or to create an incision in the tissue. In this implementation, an attachment portion 67 on a proximal end of the shaft 54 can be configured to allow for removable attachment of the shaft 54 and the end effector 60 to the handle assembly 52. In particular, mating features 68 of the attachment portion 67 can mate to complementary mating features 71 of the handle assembly 52. The mating features 68, 71 can be configured to couple together via, e.g., a snap fit coupling, a bayonet type coupling, etc., although any number of complementary mating features and any type of coupling can be used to removably couple the shaft 54 to the handle assembly 52. Although the entire shaft 54 of the illustrated implementation is configured to be detachable from the handle assembly 52, in some implementations, the attachment portion 67 can be configured to allow for detachment of only a distal portion of the shaft 54. Detachable coupling of the shaft 54 and/or the end effector 60 can allow for selective attachment of a desired end effector 60 for a particular procedure, and/or for reuse of the handle assembly 52 for multiple different procedures.

The handle assembly 52 can have one or more features thereon to manipulate and operate the end effector 60. By way of non-limiting example, a rotation knob 72 mounted on a distal end of the handle assembly 52 can facilitate rotation of the shaft 54 and/or the end effector 60 with respect to the handle assembly 52. The handle assembly 52 can include clamping components as part of a clamping system actuated by a movable trigger 74 and firing components as part of a firing system that can also be actuated by the trigger 74. Thus, in some implementations, movement of the trigger 74 toward a stationary handle 70 through a first range of motion can actuate clamping components to cause the opposed jaws 62, 64 to approximate toward one another to a closed position. In some implementations, only one of the opposed jaws 62, 24 can move to move the jaws 62, 64 to the closed position. Further movement of the trigger 74 toward the stationary handle 70 through a second range of motion can actuate firing components to cause the ejection of the staples from the staple cartridge 66 and/or the advancement of a knife or other cutting element (not shown) to sever tissue captured between the jaws 62, 64.

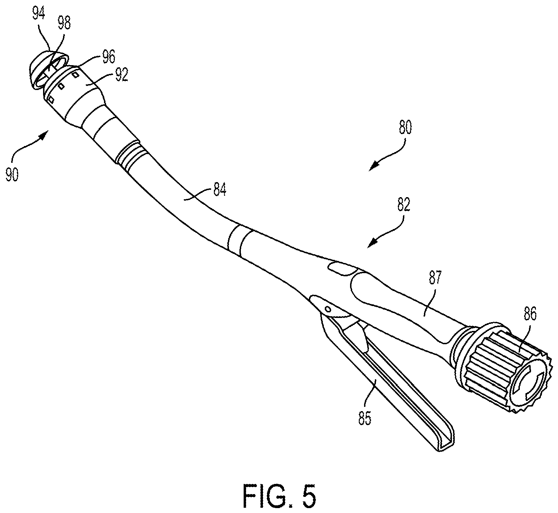

One example of a surgical instrument in the form of a circular surgical stapler 80 is illustrated in FIG. 5. The stapler 80 can generally be configured and used similar to the linear staplers 10, 50 of FIGS. 1 and 4, but with some features accommodating its functionality as a circular stapler. Similar to the surgical instruments 10, 50, the surgical instrument 80 includes a handle assembly 82 with a shaft 84 extending distally therefrom and having an end effector 90 on a distal end thereof for treating tissue. The end effector 90 can include a cartridge assembly 92 and an anvil 94, each having a tissue-contacting surface that is substantially circular in shape. The cartridge assembly 92 and the anvil 94 can be coupled together via a shaft 98 extending from the anvil 94 to the handle assembly 82 of the stapler 80, and manipulating an actuator 85 on the handle assembly 82 can retract and advance the shaft 98 to move the anvil 94 relative to the cartridge assembly 92. The anvil 94 and cartridge assembly 92 can perform various functions and can be configured to capture tissue therebetween, staple the tissue by firing of staples from a cartridge 96 of the cartridge assembly 92 and/or can create an incision in the tissue. In general, the cartridge assembly 92 can house a cartridge containing the staples and can deploy staples against the anvil 94 to form a circular pattern of staples, e.g., staple around a circumference of a tubular body organ.

In one implementation, the shaft 98 can be formed of first and second portions (not shown) configured to releasably couple together to allow the anvil 94 to be detached from the cartridge assembly 92, which may allow greater flexibility in positioning the anvil 94 and the cartridge assembly 92 in a body of a patient. For example, the first portion of the shaft 98 can be disposed within the cartridge assembly 92 and extend distally outside of the cartridge assembly 92, terminating in a distal mating feature. The second portion of the shaft 98 can be disposed within the anvil 94 and extend proximally outside of the cartridge assembly 92, terminating in a proximal mating feature. In use, the proximal and distal mating features can be coupled together to allow the anvil 94 and cartridge assembly 92 to move relative to one another.

The handle assembly 82 of the stapler 80 can have various actuators disposed thereon that can control movement of the stapler. For example, the handle assembly 82 can have a rotation knob 86 disposed thereon to facilitate positioning of the end effector 90 via rotation, and/or the trigger 85 for actuation of the end effector 90. Movement of the trigger 85 toward a stationary handle 87 through a first range of motion can actuate components of a clamping system to approximate the jaws, i.e. move the anvil 94 toward the cartridge assembly 92. Movement of the trigger 85 toward the stationary handle 87 through a second range of motion can actuate components of a firing system to cause the staples to deploy from the staple cartridge assembly 92 and/or cause advancement of a knife to sever tissue captured between the cartridge assembly 92 and the anvil 94.

The illustrated examples of surgical stapling instruments 10, 50, 80 provide only a few examples of many different configurations, and associated methods of use, that can be used in conjunction with the disclosures provided herein. Although the illustrated examples are all configured for use in minimally invasive procedures, it will be appreciated that instruments configured for use in open surgical procedures, e.g., open linear staplers as described in U.S. Pat. No. 8,317,070 entitled "Surgical Stapling Devices That Produce Formed Staples Having Different Lengths" and filed February 28, 2007, can be used in conjunction with the disclosures provided herein. Greater detail on the illustrated examples, as well as additional examples of surgical staplers, components thereof, and their related methods of use, are provided in U.S. Pat. Pub. No. 2015/0277471 entitled "Systems And Methods For Controlling A Segmented Circuit" and filed March 26, 2014, U.S. Pat. Pub. No. 2013/0256377 entitled "Layer Comprising Deployable Attachment Members" and filed February 8, 2013, U.S. Pat. No. 8,393,514 entitled "Selectively Orientable Implantable Fastener Cartridge" and filed September 30, 2010, U.S. Pat. No. 8,317,070 entitled "Surgical Stapling Devices That Produce Formed Staples Having Different Lengths" and filed February 28, 2007, U.S. Pat. No. 7,143,925 entitled "Surgical Instrument Incorporating EAP Blocking Lockout Mechanism" and filed June 21, 2005, U.S. Pat. Pub. No. 2015/0134077 entitled "Sealing Materials For Use In Surgical Stapling" and filed November 8, 2013, entitled "Sealing Materials for Use in Surgical Procedures, and filed on November 8, 2013, U.S. Pat. Pub. No. 2015/0134076, entitled "Hybrid Adjunct Materials for Use in Surgical Stapling," and filed on November 8, 2013, U.S. Pat. Pub. No. 2015/0133996, entitled "Positively Charged Implantable Materials and Method of Forming the Same," and filed on November 8, 2013, U.S. Pat. Pub. No. 2015/0129634, entitled "Tissue Ingrowth Materials and Method of Using the Same," and filed on November 8, 2013, U.S. Pat. Pub. No. 2015/0133995, entitled "Hybrid Adjunct Materials for Use in Surgical Stapling," and filed on November 8, 2013, U.S. Pat. Pub. No. 2015/0272575, entitled "Surgical Instrument Comprising a Sensor System," and filed on March 26, 2014, and U.S. Pat. Pub. No. 2015/0351758, entitled "Adjunct Materials and Methods of Using Same in Surgical Methods for Tissue Sealing," and filed on June 10, 2014.

### IMPLANTABLE ADJUNCTS

As indicated above, various implantable adjuncts are provided for use in conjunction with surgical stapling instruments. The adjuncts can have a variety of configurations, and can be formed from various materials. In general, an adjunct can be formed from one or more of a film, a foam, an injection molded thermoplastic, a vacuum thermoformed material, a fibrous structure, and hybrids thereof. The adjunct can also include one or more biologically-derived materials and one or more drugs. Each of these materials is discussed in more detail below.

An adjunct can be formed from a foam, such as a closed-cell foam, an open-cell foam, or a sponge. An example of how such an adjunct can be fabricated is from animal derived collagen, such as porcine tendon, that can then be processed and lyophilized into a foam structure. Gelatin can also be used and processed into a foam. Examples of various foam adjuncts are further described in previously mentioned U.S. Pat. No. 8,393,514 entitled "Selectively Orientable Implantable Fastener Cartridge" and filed September 30, 2010.

An adjunct can also be made from a film formed from any suitable material or a combination of materials discussed below. The film can include one or more layers, each of which can have different degradation rates. Furthermore, the film can have various regions formed therein, for example, reservoirs that can releasably retain therein one or more medicants in a number of different forms. The reservoirs having at least one medicant disposed therein can be sealed using one or more different coating layers which can include absorbable or non-absorbable polymers. The film can be formed in various ways. For example, it can be an extruded or a compression molded film. The medicants can also be adsorbed onto the film or bound to the film via non-covalent interactions such as hydrogen bonding.

An adjunct can also be formed from injection molded thermoplastic or a vacuum thermo formed material. Examples of various molded adjuncts are further described in U.S. Pat. Pub. No. 2013/0221065 entitled "Fastener Cartridge Comprising A Releasably Attached Tissue Thickness Compensator" and filed February 8, 2013. The adjunct can also be a fiber-based lattice which can be a woven fabric, knitted fabric or non-woven fabric such as a melt-blown, needle-punched or thermal-constructed loose woven fabric. An adjunct can have multiple regions that can be formed from the same type of lattice or from different types of lattices that can together form the adjunct in a number of different ways. For example, the fibers can be woven, braided, knitted, or otherwise interconnected so as to form a regular or irregular structure. The fibers can be interconnected such that the resulting adjunct is relatively loose. Alternatively, the adjunct can include tightly interconnected fibers. The adjunct can be in a form of a sheet, tube, spiral, or any other structure that can include compliant portions and/or more rigid, reinforcement portions. The adjunct can be configured such that certain regions thereof can have more dense fibers while others have less dense fibers. The fiber density can vary in different directions along one or more dimensions of the adjunct, based on an intended application of the adjunct. The adjunct can be formed from woven, knitted, or otherwise interconnected fibers, which allows the adjunct to be stretched. For example, the adjunct can be configured to stretch in a direction along its longitudinal axis and/ or in a lateral direction that is perpendicular to the longitudinal axis. While being stretchable in at least two dimensions (e.g., X and Y directions), the adjunct can provide reinforcement along its thickness (e.g., a Z direction) such that it stretches but resists tearing and pull-through by the staples. Non-limiting examples of adjuncts that are configured to be implanted such that they can stretch with the tissue are described in the above-mentioned U.S. Pat. Pub. No. 2016/0089142 entitled "Method for Creating a
Flexible Staple Line," filed on September 26, 2014.

The adjunct can also be a hybrid construct, such as a laminate composite or melt-locked interconnected fiber. Examples of various hybrid construct adjuncts are further described in U.S. Pat. No. 9,282,962 entitled "Adhesive Film Laminate" and filed February 8, 2013, and in U.S. Pat. No. 7,601,118 entitled "Minimally Invasive Medical Implant And Insertion Device And Method For Using The Same" and filed September 12, 2007.

The adjuncts in accordance with the described techniques can be formed from various materials. The materials can be used in various embodiments for different purposes. The materials can be selected in accordance with a desired therapy to be delivered to tissue so as to facilitate tissue in-growth. The materials can include bioabsorbable and biocompatible polymers, including homopolymers and copolymers. Bioabsorbable polymers can be absorbable, resorbable, bioresorbable, or biodegradable polymers. An adjunct can also include active agents, such as active cell culture (e.g., diced autologous tissue, agents used for stem cell therapy (e.g., Biosutures and Cellerix S.L.), hemostatic agents, and tissue healing agents.

The adjuncts can releasably retain therein at least one medicant that can be selected from a large number of different medicants. Medicants include, but are not limited to, drugs or other agents included within, or associated with, the adjuncts that have a desired functionality. The medicants include, but are not limited to, for example, antimicrobial agents such as antibacterial and antibiotic agents, antifungal agents, antiviral agents, anti-inflammatory agents, growth factors, analgesics, anesthetics, tissue matrix degeneration inhibitors, anticancer agents, hemostatic agents, and other agents that elicit a biological response. The adjuncts can also be made from or include agents that enhance visibility during imaging, such as, for example, echogenic materials or radio-opaque materials.

Examples of various adjuncts and various techniques for releasing medicants from adjuncts are further described in U.S. Pat. App. No. 14/840,613 entitled "Medicant Eluting Adjuncts and Methods of Using Medicant Eluting Adjuncts" and filed August 31, 2015.

### IMPLEMENTATIONS

An adjunct can be releasably retained on a jaw of an end effector for a surgical tool using various retaining or attachment features. In some implementations, the attachment feature can be disposed over the adjunct material and it can be releasably coupled to the jaw on which the adjunct is disposed. The adjunct material can be separated from the jaw in a suitable way. For example, the attachment feature retaining the adjunct on the jaw can be cut by a suitable cutting element (e.g., a knife) as the cutting element translates distally to cut tissue retained between the jaws. In some embodiments, when staples are ejected from staple-holding cavities of a cartridge, the staples cause the adjunct material to be separated from the jaw. For example, the force with which the staples are ejected can cause the adjunct material to be disengaged from the jaw. Additionally or alternatively, one or more portions of the attachment feature, or the entire attachment feature can be biodegradable and/or bioabsorbable, and the attachment feature or a portion thereof can therefore remain with the adjunct material when it is transferred to a treatment site in a patient.

In some implementations, an adjunct material is releasably retained on a jaw of an end effector using an attachment feature having a retaining filament. The retaining filament can have an intermediate portion and first and second ends disposed on opposed sides of the intermediate portion. Each of the first and second ends can have a respective end feature configured to mate with the jaw. To retain the adjunct material on the jaw, the attachment feature can be arranged such that at least a part of its intermediate portion is disposed over the adjunct material and such that the first and second ends are spaced apart. The first and second ends can be disposed on a side of the jaw opposed to the tissue-facing surface, and they can be spaced apart across a cutting element channel of the jaw. It should be appreciated that, the "first" and "second," as used herein in connection with the ends of the retaining filament or in connection with any other elements, features, or portions described herein, are used for the description purposed only, and not to indicate any particular order.

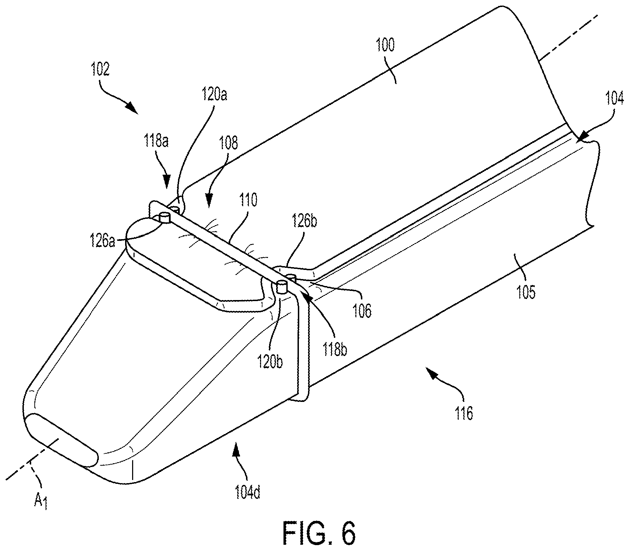

FIGS. 6-8 illustrate an implementation of an adjunct material 100 releasably retained on a jaw of an end effector 102 in accordance with the described techniques. The end effector 102, shown only partially in FIG. 6, can be used with any suitable surgical instrument, for example, a linear surgical stapler (e.g., stapler 10 in FIG. 1, stapler 50 in FIG. 4, or any other surgical stapler) that is suitable for use with at least one adjunct. The end effector 102 can be coupled to a distal end of a shaft of the surgical stapler (not shown). The jaw of the end effector 102 is in the form of a cartridge body 104 with a plurality of staple cavities configured to seat staples therein. The staple cavities, which are obscured by the adjunct material 100 in FIG. 6, open on a tissue-facing surface 106 of the cartridge body 104. The cartridge body 104 can be or can have a removable and replaceable cartridge retained therein, or, in some embodiments, the cartridge body 104 can be part of a disposable loading unit removably coupled to an elongate shaft of a surgical instrument.

Although not shown in FIG. 6, the end effector 102 also has an anvil opposing the cartridge body 104, with a plurality of staple forming cavities formed on a tissue-facing surface thereof. It should be appreciated that the adjunct material 100 is shown in FIG. 6 to be releasably retained on the jaw in the form of the cartridge body 104 by way of example only, as an adjunct material can be retained in a similar manner on an anvil of an end effector as well.

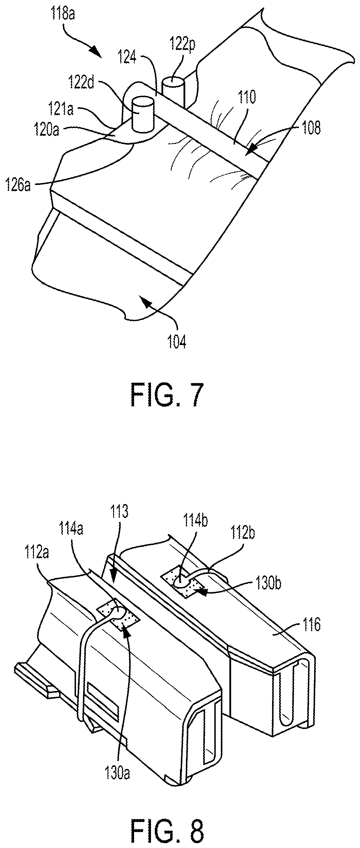

As shown in FIG. 6, the adjunct material 100 is retained on the cartridge body 104 using an attachment feature in the form of an elongate retaining filament 108. In this implementation, the retaining filament 108 has an intermediate portion 110 and first and second ends 112a, 112b disposed on opposed sides of the intermediate portion 110, which are shown in FIG. 8. As shown in FIG. 6, the retaining filament 108 is arranged on the cartridge body 104 such that at least a part of the intermediate portion 110 is disposed over the adjunct material 100. The intermediate portion 110 of the retaining filament 108 also encompasses opposed sides walls of the cartridge body 104, one of which, side wall 105, is shown in FIG. 6.

In this implementation, the intermediate portion 110 of the retaining filament 108 extends over the side walls of the cartridge body 104 such that the first and second ends 112a, 112b are spaced apart. In particular, the first and second ends 112a, 112b are disposed on a side 116 (back side when the end effector's jaws are closed) of the cartridge body 104 that is opposed to the tissue-facing surface 106 thereof, as shown in FIG. 8. The first and second ends 112a, 112b can be spaced apart across a cutting element channel 113 of the cartridge body 104 extending longitudinally across a mid-portion of the cartridge body 104.

At least a portion or the entirety of the retaining filament 108 can be removably attached to the cartridge body 104 in a variety of different ways. For example, in the illustrated embodiments, the retaining filament 108 can be attached to the cartridge body 104 using a hotmelt adhesive or any other suitable type of adhesive or glue material. The adhesive can be, for example, polydioxanone (PDO) that can function as an adhesive when heated. Additionally or alternatively, cyanoacrylates or UV curing adhesives can be used.

The retaining filament 108 can be used to retain the adjunct material 100 on the jaw 104 using one or more suitable features that can be formed on the jaw and/or on the adjunct material. In the illustrated example, as shown in FIGS. 6 and 7, the cartridge body 104 has retaining members 118a, 118b disposed at opposed sides 120a, 120b of the tissue-facing surface 106 in proximity to the respective edges of the tissue-facing surface 106. Each of the retaining members 118a, 118b can be in the form of a pair of adjacent posts, each of which is configured to engage at least a part of the intermediate portion 110 of the retaining filament 108. FIG. 7, showing the retaining member 118a, illustrates a pair of adjacent posts 122d, 122p disposed on the side 120a of the tissue-facing surface 106 of the cartridge body 104, in proximity to the edge 121a of the tissue-facing surface 106. The distance between the adjacent posts 122d, 122p is such that the posts 122d, 122p engage the part of the intermediate portion 110, indicated in FIG. 7 as the part 124 that extends between the posts 122d, 122p. The part 124 of the retaining filament 108 passing between the posts 122d, 122p can be engaged with the posts 122d, 122p via interference fit. As shown in FIG. 6, similar to the retaining member 118a (FIG. 7), the retaining member 118b formed on the opposite side 120b of the tissue-facing surface 106 can be in the form of a pair of adjacent posts.

In some embodiments, the posts in a pair of posts formed on the tissue-facing surface of the jaw can be spaced from one another such that they have a portion of the retaining filament (e.g., an intermediate portion) passing therethrough without being engaged between the posts. In this way, the posts ensure that the retaining filament is positioned as desired and prevent the retaining filament from sliding proximally or distally. Additionally or alternatively, the retaining filament can be retained over the jaw's surface using adhesive which can be used to couple one or more portions of the retaining filament to the jaw.

As shown in FIGS. 6 and 7, the posts 122d, 122p are disposed along the edge 121a such that the post 122d is more distal (e.g., closer to the distal end 104d of the cartridge body 104) than the adjacent post 122p. Also, in the illustrated exemplary implementation, the posts 122d, 122p are offset by the same or substantially the same distance from the edge of the cartridge body 104 such that the posts 112d, 112p are disposed along the same line parallel to a longitudinal axis A1 of the cartridge body 104. It should be appreciated, however, that the posts 122d, 122p can be disposed on the tissue-facing surface 106 in other ways. Moreover, more than two posts or other retaining elements can be formed on the tissue-facing surface 106 for engaging the part of the intermediate portion 110. Furthermore, in some embodiments, the retaining member can be in the form of a single element formed on the tissue-facing surface 106, the single element having one or more prongs, arms, or other retaining elements configured to frictionally engage an attachment features therebetween. As another options, different types of retaining members can be formed on opposed sides of the tissue-facing surface of the jaw of an end effector.

The adjunct material 100 releasably retained on the tissue-facing surface 106 of the jaw 104 can have a variety of different configurations. As shown in FIG. 6, the adjunct material 100 is generally rectangular and it is sized such that its width is substantially the same as the width of the tissue-facing surface 106. The length of the adjunct material 100 can be greater than that of the tissue-facing surface 106 - as shown in FIG. 6, the adjunct 100 extends distally beyond the tissue-facing surface 106. This makes it possible to retain the adjunct 100 using the retaining members 118a, 118b formed at close proximity to the distal end of the tissue-facing surface 106.

The adjunct material 100 can have features that facilitate its temporary engagement with the cartridge body 104. Thus, as shown in FIGS. 6 and 7, the adjunct material 100 has cut-outs 126a, 126b formed on opposite sides thereof such that the retaining members 118a, 118b are disposed within the cut-outs 126a, 126b, respectively.

In the illustrated implementation, the cartridge body 104 can have, in addition to the retaining members 118a, 118b, other features that facilitate releasable attachment of the adjunct material 100 to the cartridge body 104. Thus, as shown in FIG. 8, the side 116 of the cartridge body 104 opposed to the tissue-facing surface 106 has roughened portions 130a, 130b which the first and second ends 112a, 112b of the retaining filament 108 engage frictionally. The first and second ends 112a, 112b can have end features in the form of flattened leaf portions or members 114a, 114b (or other types of end features) formed thereon that are configured to frictionally engage with the roughened portions 130a, 130b, respectively. FIG. 8 illustrates that the first and second ends 112a, 112b with the leaf members 114a, 114b are spaced apart across the cutting element channel 113 of the cartridge body 104.

The leaf portions can be formed in a variety of ways. For example, they can be preformed elements with a texture and/or surface features that allow them to engage the roughened portions. In some embodiments, however, the leaf portions can be formed by heat-pressing or otherwise processing end portions of the retaining filament to flatten them and thus form leaf-like ends. In such embodiments, the end portions of the retaining filament can be caused to deform and to be "pressed" onto the roughened portions. The heat pressing can be performed in a variety of ways, for example, by pressing the end portion of the retaining filament (which can be made from a thermoplastic material) into the roughened portions with a heated iron device so that the thermoplastic material is heated and cooled after mechanically "locking" into the roughened portions of the jaw.

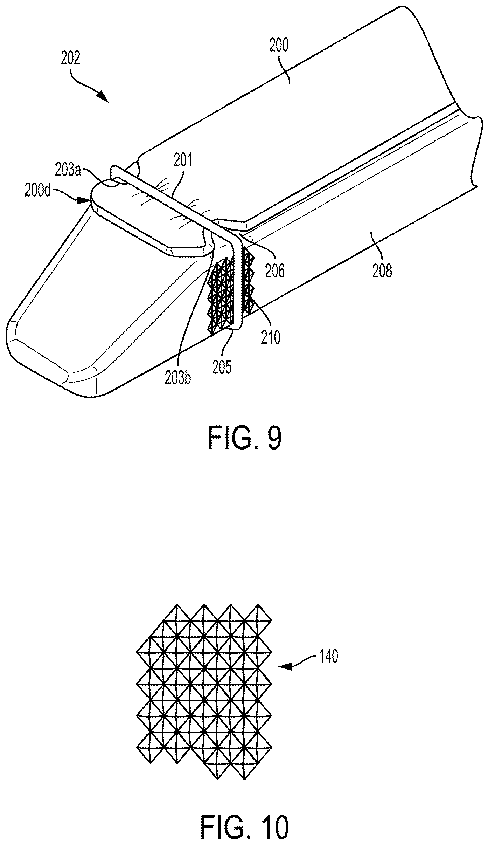

The roughened portions 130a, 130b can be created in many different ways. In the illustrated implementation, they are formed by modifying a surface texture of a portion of the cartridge's side 116. For example, the portions of the cartridge's side 116 can be knurled or otherwise roughened to create the roughened portions 130a, 130b of a desired size at appropriate locations. FIG. 10 illustrates an example of a knurled or roughened portion 140, which can be any of the roughened portions 130a, 130b. As shown in FIG. 10, the roughened portion 140 has a regular pattern of small four-sided pyramids, though the regular pattern can be formed from elements of any other type(s). Also, the roughened portion 140 can be an irregular rough portion configured to engage with a portion of an attachment feature, e.g., the first and second ends 112a, 112b with the leaf portions 114a, 114b or with otherwise shaped elements.

The roughened portions 130a, 130b can be created in any suitable manner. For example, they can be created by laser etching, chemically etching, heat altering (flame treated, heat pressed/stamped, etc.) or mechanically etching (grinding, sand blasting, CO₂ blasting, etc.) the surface of the jaw. In some implementations, the roughened portions 130a, 130b can be created by depositing certain materials (e.g., pressure-sensitive adhesives) over the surface of the cartridge body 104, or by otherwise modifying the texture of a portion of the side 116 of the cartridge body 104.

Accordingly, in the example of FIGS. 6-8, a mid-portion of the intermediate portion 110 of the retaining filament 108 extends over the adjunct material 100 and portions extending from both sides of the mid-portion encompass the opposed side walls of the cartridge body 104 such that the spaced apart first and second ends 112a, 112b are disposed over the side 116 opposed to the tissue-facing surface 106. The adjunct material 100 is releasably retained on the cartridge body 104 using the pairs of the adjacent posts 118a, 118b and the roughened portions 130a, 130b.

The retaining filament 108 can have any form and it can be made from any suitable materials. For example, it can be in the form of a suture, wire, cable, strap, or in any other form. It can be made from any suitable absorbable or non-absorbable polymers, examples of which include polyglactin, polyglycolic acid, catgut, polyglecaprone, polydioxanone, etc. Non-limiting examples of non-dissolvable suture materials include polypropylene, polyamide, polyester, silk, etc. In some embodiments, the retaining filament 108 can be formed from at least partially resilient and/or pliable material such that it can be manipulated to conform to a shape of a body of a jaw which it partially encompasses.

The end features in the form of leaf members 114a, 114b formed on the first and second ends 112a, 112b of the retaining filament 108, which can be formed integrally with the retaining filament 108 or can be coupled to the retaining filament 108 in a suitable manner, can also be formed from any suitable material(s), including the absorbable or non-absorbable polymers mentioned above. As mentioned above, each leaf member can be formed by pressing (e.g., heat-pressing) a portion of the retaining filament onto the jaw's surface. In some implementations, the leaf members 114a, 114b, or other members configured to engage the roughened portions 130a, 130b, can be made from a relatively rigid material. The leaf members 114a, 114b can have surface features (e.g., ridges, hooks, barbs, or any other protruding features) formed thereon that allow the leaf members 114a, 114b to frictionally and removably engage with the roughened portions 130a, 130b. Furthermore, in some embodiments, the leaf members 114a, 114b can be formed from at least partially magnetic material such that they can be magnetically retained on the roughened portions 130a, 130b. Also, in other embodiments, the leaf members 114a, 114b can be coupled to the roughened portions 130a, 130b using a polymer-based magnetic gels, or in other manner.

The roughened portions can be formed at any one or more portions of the jaw that can be engaged with a retaining filament. In the implementations described above, the roughened portions can be formed on a side that is opposite to a tissue-facing surface, such as an upper side of an anvil or a backside of a cartridge. In some implementations, additionally or alternatively, regardless of their configuration(s) and the way in which they are formed, one or more roughened portions can be formed on various others portions of a jaw of an end effector. For example, FIG. 9 shows an example of a jaw 202 of an end effector having an adjunct material 200 releasably retained thereon using an attachment feature. The attachment feature is in the form of a retaining filament 201 engaged with roughened portions formed on opposed side walls of the jaw 202. The engagement can be chemical (e.g., using an adhesive), frictional or other mechanical engagement, or any other type of engagement, including a combination of different ways. In some embodiments, a heat pressing approach can be used to allow mechanical interlocks of complementary matches of the surface geometry.

In this example, the jaw 202 is in the form of a cartridge body which can be similar to the cartridge body 104 in FIGS. 6-8. The adjunct material 200, which can be similar to the adjunct material 100 (FIGS. 6-8) is disposed on a tissue-facing surface 206 of the jaw 202. Similar to the adjunct material 100, the adjunct material 200 has cut-outs 203a, 203b formed in proximity to a distal end 200d thereof on both long sides of the adjunct 200.

As shown in FIG. 9, side walls of the cartridge body 104 can be roughened, which can be done in a manner similar to a manner in which the roughened portions 130a, 130b are created on the cartridge body 104. FIG. 9 illustrates that a portion of one of the side walls (208) of the jaw 202 is roughened to thus form a roughened portion 210. A portion 205 of the retaining filament 201, encompassing the side wall of the jaw 202, frictionally engages the roughened portion 210 and thereby releasably retains the adjunct material 200 on the jaw 202. The opposite side of the jaw 202, which is not shown in FIG. 9, can have similar roughened portion which the retaining filament 201 encompassing that side wall frictionally engages. The jaw 202 can also have one or more roughened portions on a side thereof that is opposed to its tissue-facing surface 206, and such roughened portions can be similar to the roughened portions 130a, 130b of the jaw 204 shown in FIG. 8. Also, although the jaw 202 is shown without any other retaining features that can be used to temporarily engage the retaining filament 201 with the jaw 202, it should be appreciated that the jaw 202 can have other retaining features. For example, the jaw 202 can include retaining members similar to the retaining members 118a, 118b in the form of pairs of adjacent posts (FIGS. 6 and 7), roughened portions on the backside side of the jaw and/or any other retaining features.

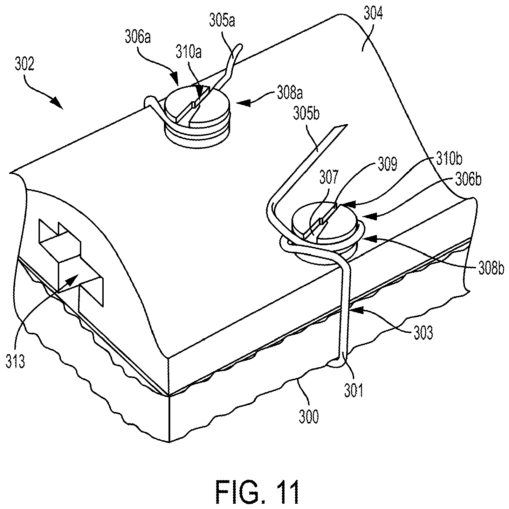

FIG. 11 illustrates another implementation of retaining members formed on a jaw 302 of an end effector having an adjunct material 300 releasably retained thereon. In this example, a side 304 (upper side) of the jaw 302 that is opposite to a tissue-facing surface of the jaw 302 is shown (e.g., the side 116 of the jaw 102 in FIG. 8). In FIG. 11, the jaw 302 is in the form of an anvil. However, as a person skilled in the art will appreciate, an end effector's jaw having retaining members as described in connection with FIG. 11 can be a cartridge body.

The adjunct material 300 is releasably retained on the jaw 302 using an attachment feature in the form of a retaining filament 301, portions of which are shown in FIG. 11. The retaining filament 301 has an intermediate portion a part of which is disposed over the adjunct 300 (not shown in FIG. 11) and other parts of which (e.g., 303) encompass the jaw's side wall.

The retaining filament 301 also has first and second ends 305a, 305b disposed on both sides of the intermediate portion adjacent to the parts of the retaining filament 301 encompassing the side wall of the jaw 302. In the example shown in FIG. 11, the first and second ends 305a, 305b are disposed on the upper side 304 of the jaw 302 and retained on that side via the respective retaining members. The retaining members are spindle-type retaining members 306a, 306b disposed on opposed sides of the surface of the jaw's side 304 in proximity to the edges of the jaw 302. Each of the spindle-type retaining members 306a, 306b is a generally cylindrical member having a relatively small diameter and height. For example, in at least one embodiment, the diameter of the retaining members 306a, 306b
can be about 1,524 mm (0.060 inches), and its height can be about 0. 635 mm (0.025 inches). Regardless of its size, each of the of the spindle-type retaining members 306a, 306b has a radial recess formed in the member's side wall around the entire circumference of the wall. Thus, FIG. 11 shows that the retaining members 306a, 306b have radial recesses 308a, 308b, respectively. The retaining members 306a, 306b also have respective holding notches or recesses 310a, 310b that are formed along each of the member's diameter on the top side of that member.

It should be appreciated that the spindle-type retaining members 306a, 306b are shown in FIG. 11 by way of example only, and that any other features can be used to couple the retaining filament to the jaw. For example, in some embodiments, the jaw (a cartridge or an anvil) can be configured to decrease in width so as to "grab" the filament. As another example, the jaw can have a groove with a cam feature, a post with an adjacent cam feature, or any other feature(s) configured to retain the retaining filament which can be a rope, wire, suture, thread, or any other element.

The radial recesses 308a, 308b and the holding recesses 310a, 310b are used to retain therein a portion of the at least one of the first and second ends 305a, 305b. In particular, as shown in FIG. 11, the retaining member 306a has the first end 305a of the retaining filament 301 receiving within the radial recess 308a such that the first end 305a is wrapped around the retaining member 306a. After the first end 305a encircles the retaining member 306a at least once, a portion of the first end 305a is fittingly received through the top recess 310a of the retaining member 306a. In this way, the first end 305a of the retaining filament 301 engages with the retaining member 306a.

The holding recesses 310a, 310b can have a configuration that facilitates retention of a portion of the retaining filament 301. For example, the holding recess 310b of the retaining member 306b (shown in FIG. 11 free of the retaining filament for the illustration purposes only) has a first wider portion 307 and a second, narrower portion 309 extending from the first portion 307. The holding recess 310a of the retaining member 306a is configured in a similar manner. In use, after a portion of the second end 305b of the retaining filament 301 is wrapped around the radial recess 308b, the first portion 307 receives therein another portion of the second end 305b and this filament's portion is then received through the narrower portion 309 of the holding recess 310b so as to be fittingly retained therein. Thus, in use, the second end 305b can be slightly stretched and passed through the holding recess 310b so as to be retained within the recess. The first end 305a is engaged with the holding recess 310a in a similar manner and is shown in FIG. 11 as being wrapped around the radial recess 308a and retained within the holding recess 310a. In this way, both ends of the retaining filament 301 are temporarily engaged with the jaw 302.

As in the examples above, the retaining filament 301 can be disengaged from the jaw 302 when a knife or other cutting element traverses a cutting element channel 313 and thereby cuts a portion of the retaining filament 301 disposed over the adjunct material 300. The rest of the retaining filament 301 remains with the jaw 302.

It should be appreciated that, regardless of the specific configurations of attachment features (e.g., retaining filaments or other features) described herein, jaw(s) of an end effector, the attachment features, and one or more adjunct materials are configured such that the jaw having one or more adjunct materials releasably retained thereon using one or more attachment features fits within a trocar. In some embodiments, one or more attachment features (e.g., posts or other features) can be formed at a bottom of one or more recesses formed in a jaw, such that the attachment features do not affect the overall size of the jaw. The adjunct material(s) are configured to be retained on the jaw in a manner that does not interfere with normal manipulations and operation of the jaw.

An adjunct material can be releasably retained on an end effector's jaw using various other types of attachment features in the form of a retaining filament. For example, in some implementations, an attachment feature has an intermediate portion and first and second ends with deformable elements. The deformable elements can be configured such that, when the attachment feature is disposed over an adjunct material placed on the jaw of an end effector, the deformable elements reversibly deform and change their configuration as they are received within openings or recesses in the jaw. When the deformable elements are engaged with the recesses in the jaw, they at least partially adopt their non-deformed configuration to thus retain the attachment feature in place.

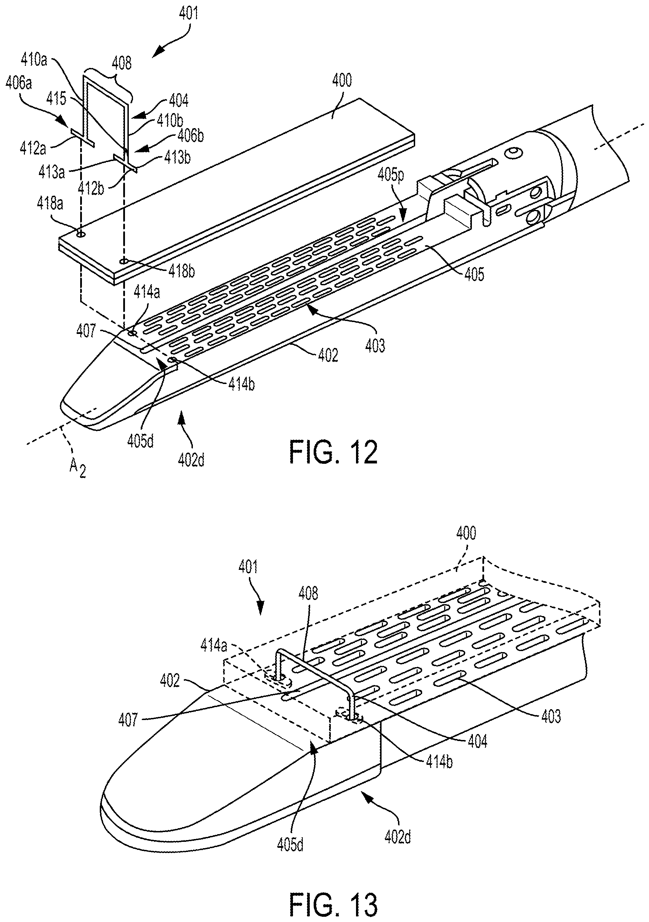

FIGS. 12 and 13 illustrate an example, not forming part of the invention, of an adjunct material 400 configured to be releasably retained on a jaw 402 of an end effector using an attachment feature 401. The attachment feature 401 has an intermediate portion 404 and first and second ends 406a, 406b. The intermediate portion 404, in turn, includes a mid-portion 408 and first and second arm portions 410a, 410b extending from opposite sides of the mid-portion 408 and terminating at the first and second ends 406a, 406b. As shown in FIGS. 12 and 13, the first and second ends 406a, 406b have deformable elements 412a, 412b configured to be reversibly deform. In this example, the deformable elements 412a, 412b are in the form of t-shaped barb members. However, it should be appreciated that the deformable elements 412a, 412b can have any other suitable configurations. For example, they can be configured as Christmas tree-type, umbrella-like, or any other types of deformable elements configured to be used to retain an adjunct material on a jaw as discussed in more detail below.

The jaw 402 can be configured in many different ways. In the example of FIGS. 12 and 13, the jaw 402 is in the form of a cartridge body or cartridge having a plurality of staple cavities 403 configured to seat staples therein, the staple cavities opening on a tissue-facing surface 405 of the cartridge 402. As shown in FIG. 12, the staple cavities 403 form three rows on both sides of a cutting element channel 407 extending through a mid-portion of the cartridge 402 along a longitudinal axis A2 thereof. It should be appreciated, however, that any suitable number of the staple cavities 403 can have any suitable pattern(s) on the tissue-facing surface 405 of the jaw 402, as the described embodiments are not limited in this respect.

The cartridge 402 can have suitable features configured to retain an adjunct material thereon. As shown in FIGS. 12 and 13, the cartridge 402 has a first pair of recesses 414a, 414b formed in the tissue-facing surface 405 thereof. The recesses of the first pair of recesses 414a, 414b are spaced from opposed edges of the tissue-facing surface 405 and are disposed on opposed sides of the cutting element channel 407 extending centrally through the cartridge 402 along the longitudinal axis A2 thereof. The first recesses 414a, 414b are formed on a distal portion 405d of the tissue-facing surface 405 that is free of the staple cavities, as shown in FIGS. 12 and 13. In some embodiments, however, the first recesses 414a, 414b can be formed within the area of the tissue-facing surface 405 having the staple cavities formed thereon.

The first recesses 414a, 414b formed in the tissue-facing surface 405 of the cartridge 402 can have many different configurations. In this example, the first recesses 414a, 414b can have a generally oval cross-sectional shape and they can be sized to allow therewithin the first and second ends 406a, 406b with the deformable elements 412a, 412b. Although not shown in FIGS. 12 and 13, the inner walls of the first recesses 414a, 414b can have a configuration and size that allow the deformable elements 412a, 412b to be at least partially deform as they are received within the recesses 414a, 414b and to then return at least in part to their non-deformable configuration to thus be retained in the recesses 414a, 414b. Furthermore, in some implementations, the first recesses 414a, 414b can be formed through the entire thickness of the jaw 402 such that the deformable elements 412a, 412b return at least in part to their non-deformable configuration on the side of the jaw 402 that is opposed to the tissue-facing surface 405 of the jaw 402.

The adjunct material 400 can have many different configurations. In the illustrated embodiment, the adjunct material 400 is generally rectangular, with its width and length generally corresponding to the width and length of the tissue-facing surface 405 of the cartridge 402. In the illustrated example, the adjunct material 400 has features configured to retain it on the cartridge 402. In particular, the adjunct material 400 has a second pair of through openings or recesses 418a, 418b formed therein. As shown schematically in FIGS. 12 and 13, the second recesses 418a, 418b are formed at locations in the adjunct material 400 that correspond to the location of the first recesses 414a, 414b formed in the tissue-facing surface 405 of the cartridge 402. Each of the second recesses 418a, 418b can have a size that is similar to sizes of the first recesses 414a, 414b, or the size of each of the second recesses 418a, 418b can be slightly smaller than that of the first recesses 414a, 414b.

In use, when the adjunct material 400 is disposed on the tissue-facing surface 405 of the cartridge 402, the adjunct's second recesses 418a, 418b align with the cartridge's first recesses 414a, 414b. In this way, the second recess 418a is disposed above and communicates with the first recess 414a, and the second recess 418b is disposed above and communicates with the first recess 414b. The retaining filament 301 is manipulated to cause its first and second ends 406a, 406b to be pushed through the adjunct's second recesses 418a, 418b and then to be allowed within the second recesses 418a, 418b in the cartridge 402. The mid-portion 408 of the retaining filament 301 is disposed over the adjunct 400, as shown in FIG. 13. The first and second arm portions 410a, 410b extend through the thickness of the adjunct 400 and can at least partially extend through the body of the cartridge 402.

FIG. 12 shows the deformable elements 412a, 412b in their natural, non-deformed state. Thus, for example, in the non-deformed state or configuration of the element 412a, its prongs 413a, 413b are perpendicular to a post 415. The deformable elements 412a, 412b can be resiliently deformable such that, as they are passed through the second recesses 418a, 418b in the adjunct 400, under the load applied thereto, they are caused to accept at least partially non-deformed state, e.g., the prongs 413a, 413b of the element 412a come closer to its post 415. In such at least partially unexpanded configuration, the elements 412a, 412b are then passed though the openings of the first recesses 414a, 414b in the jaw 402, upon which the elements 412a, 412b return at least in part to their expanded configuration, to be retained within the first recesses 414a, 414b. Thus, in the example of the element 412a, the prongs 413a, 413b move away from the post 415 to form an acute angle with the post 415 or to be disposed perpendicular thereto (if they fully return to the expanded configuration). In the implementations in which the first recesses 414a, 414b in the jaw 402 are in the form of through openings, the deformable elements 412a, 412b can expand on the surface of the jaw opposite to the tissue-facing surface 405 thereof. In this way, for example, the prongs 413a, 413b will be pressed against that surface of the jaw.

The adjunct material 400 releasably retained on the cartridge 402 can be separated from the cartridge 402 in different ways. For example, a cutting element (e.g., a knife), as it translates through the cutting element channel 407 formed centrally in the jaw 402, can cut the retaining filament 401 disposed, as shown in FIG. 13, above the channel 407.

It should be appreciated that the cartridge 102 can have other features for releasably retaining therein the adjunct material 400. For example, in some embodiments, a proximal end 405p of the tissue-facing surface 405 can include retaining members similar to the first recesses 414a, 414b. In such embodiments, the adjunct material 400 can also have openings similar to the second recesses 418a, 418b. Additionally or alternatively, other retaining features can be formed on the cartridge and/or on the adjunct.

In some embodiments, an end effector can have two separate adjunct materials releasably retained thereon. One ("first") of the separate adjunct materials can be configured to be disposed on one jaw of the end effector (e.g., a cartridge body), and another ("second") adjunct material can be configured to be disposed on another jaw of the end effector. The first and second adjunct materials have respective first and second mating features formed at proximal ends thereof. The end effector has an attachment feature formed at a proximal end thereof and configured to mate with at least one the first and second mating features of the first and second adjunct materials.

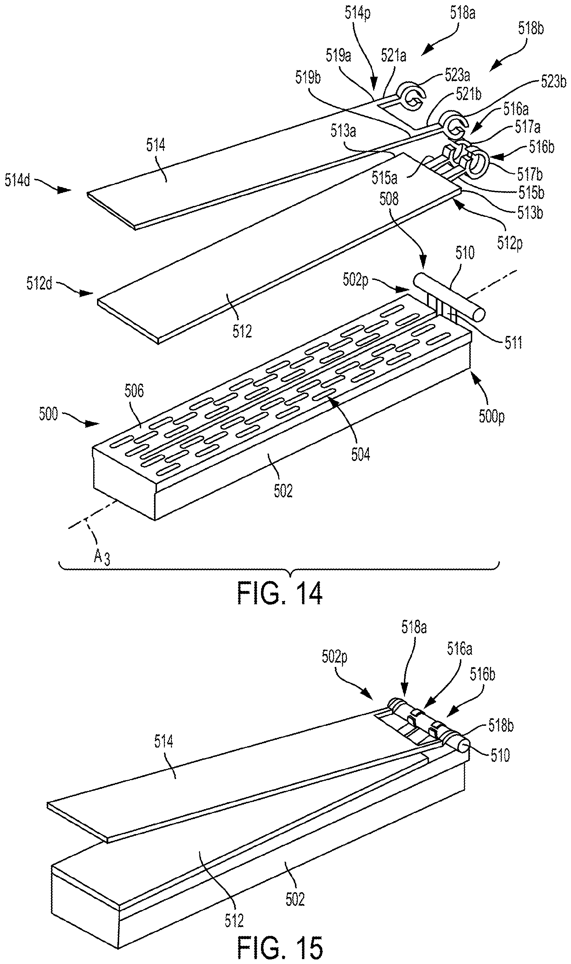

FIGS. 14 and 15 illustrate an implementation of an end effector 500, not forming part of the invention, having two separate adjunct materials releasably retained thereon. The end effector 500 can have first and second jaws configured to clamp tissue therebetween, such as a jaw having a cartridge with a plurality of staple cavities configured to seat staples therein, and another, opposing jaw having an anvil with a plurality of staple forming cavities formed on a tissue-facing surface thereof. In this example, only a portion of the end effector 500 in the form of a jaw having a cartridge body 502 (also partially shown) is illustrated.

The end effector 500 can be used with any suitable surgical instrument, for example, a linear surgical stapler (e.g., stapler 10 in FIG. 1, stapler 50 in FIG. 4, or any other surgical stapler) that is suitable for use with at least one adjunct material. The end effector 500 can be coupled to a distal end of a shaft of the surgical stapler (not shown). The cartridge body 502 has a plurality of staple cavities 504 that are configured to seat staples therein and that open on a tissue-facing surface 506 of the cartridge body 502. The cartridge body 502 can be in the form of a channel that removably and replaceably seats a cartridge therein, or the cartridge body 502 with the staples can itself be a removable and replaceable unit. Also, in some embodiments, the cartridge body 502 holding the staples can part of a disposable loading unit removably coupled to an elongate shaft of a surgical instrument.

As mentioned above, the end effector 500 has first and second adjunct materials 512, 514 configured to be releasably coupled thereto. The end effector 500 has an attachment feature configured to mate with the first and second adjunct materials 512, 514. Specifically, as shown in FIG. 14, the end effector 500 has an attachment feature 508 formed at a proximal end 500p thereof. In this implementation, the attachment feature 508 is coupled to a proximal end 502 of the cartridge body 502, though in other implementations of the present subject matter, the attachment feature 508 can be coupled to an anvil or to element(s) of the end effector that are not part of the cartridge or the anvil.

Furthermore, in the described implementation, the attachment feature 508 is in the form of a substantially cylindrical bar 510 positioned above the tissue-contacting surface 506 of the cartridge body 502 and oriented so as to be transverse to a longitudinal axis A3 of the end effector 500. The cylindrical bar 510 is coupled to a support member 511 so as to be positioned above the tissue-contacting surface 506, as shown in FIG. 14. The cylindrical bar 510 can be integrally and/or monolithically formed with the support member 511, or it can be coupled to the support member 511 in a suitable member.

The first adjunct material 512 having proximal and distal ends 512p, 512d is configured to be releasably retained on one jaw of the end effector 500, such as, in this example, the cartridge body 502. The second adjunct material 514 having proximal and distal ends 514p, 514d is configured to be releasably retained on another, opposed jaw of the end effector 500, such as an anvil which is not shown. Each of the first and second adjunct materials 512, 514 has a respective mating feature at the proximal end 512p, 514p thereof for mating with the end effector's attachment feature 508. In particular, as shown in FIG. 14, the first adjunct material 512 has a first mating feature in the form of first open-ended loop features 516a, 516b. Each of the first open-ended loop features 516a, 516b includes an arm 515a, 515b and an open-ended loop 517a, 517b. As shown in FIG. 14, the open-ended loops 517a, 517b have their gaps or open ends on the top of the loops such that the open ends face away from the cartridge body 502, in a direction substantially transverse to the longitudinal axis A3 of the end effector 500 and towards the opposed jaw (not shown). As also shown in FIG. 14, the open-ended loop features 516a, 516b of the first adjunct material 512 are formed on the proximal end 512p such that they are spaced away from opposed edges 513a, 513b of the proximal end 512p and are thus formed adjacent to one another and in proximity to a mid-portion of the proximal end 512p.

The second adjunct material 514 has a second mating feature in the form of second open-ended loop features 518a, 518b. Each of the second open-ended loop features 518a, 518b includes an arm 521a, 521b and an open-ended loop 523a, 523b. As shown in FIG. 14, the open-ended loops 523a, 523b have their gaps or open ends on the side of the loops such that the open ends face away from the cartridge body 502, in a direction substantially parallel to the longitudinal axis A3 of the end effector 500. The open-ended loop features 518a, 518b are formed on the proximal end 514p of the second adjunct material 514 such that they are adjacent to opposed edges 519a, 519b of the proximal end 514p.

The configurations of the mating features of the first and second adjunct materials 512, 514 allow the mating features to mate with the attachment feature 508 as shown in FIG. 15. The open-ended loops of the first and second open-ended loop features 516a, 516b, 518a, 518b have circumferences that are slightly undersized relative to cylindrical bar 510 such that the open-ended loops can slightly deform to encompass the bar 510. The first and second open-ended loop features 516a, 516b, 518a, 518b can be formed from at least partially resilient material such that the features can be snapped onto the bar 510 through the gaps in the open-ended loops. The non-limiting examples of the materials include polymers such as, e.g., polydioxanone (PDO), poly(glycerol sebacate) (PGS)/poly(lactic acid) (PLA), poly(glycolic acid) (PGA)/ polycaprolactone (PCL), trimethylene carbonate (TMC)/PGA, or any other suitable material or a combination of materials.

Further, the first and second open-ended loop features 516a,516b, 518a, 518b are configured to engage the bar 510 such that the first open-ended loop features 516a,516b engage the bar 510 at locations different from locations at which the second open-ended loop features 518a, 518b engage the bar 510. In particular, as shown in FIG. 15, the first open-ended loop features 516a, 516b engage the bar 510 at the locations on the bar 510 between the locations at which the second open-ended loop features 518a, 518b engage the bar 510. In this way, both the first and second open-ended loop features 516a, 516b, 518a, 518b movably engage the bar 510 to thereby engage the separate first and second adjunct materials 512, 514 with the end effector 500. In some embodiments, the open-ended loops can be segmented in such a way that the opposing sides are not directly opposite to one another. For example, the loops can be staggered.

In some implementations of the current subject matter, one or both of the first and second adjunct materials 512, 514 can have additional attachment features configured to releasably couple the adjunct materials 512, 514 with the respective opposed jaws of the end effector. For example, one or more portions of the second adjunct material 514 configured to be disposed on the anvil can be releasably coupled to the anvil using an adhesive material. Other attachment features can be formed on the second adjunct material 514 and/or on the anvil in addition to the second open-ended loop features 518a, 518b. Also, in some implementations, the first adjunct material 512 can be coupled to the cartridge body 502 using one or more additional attachment features.

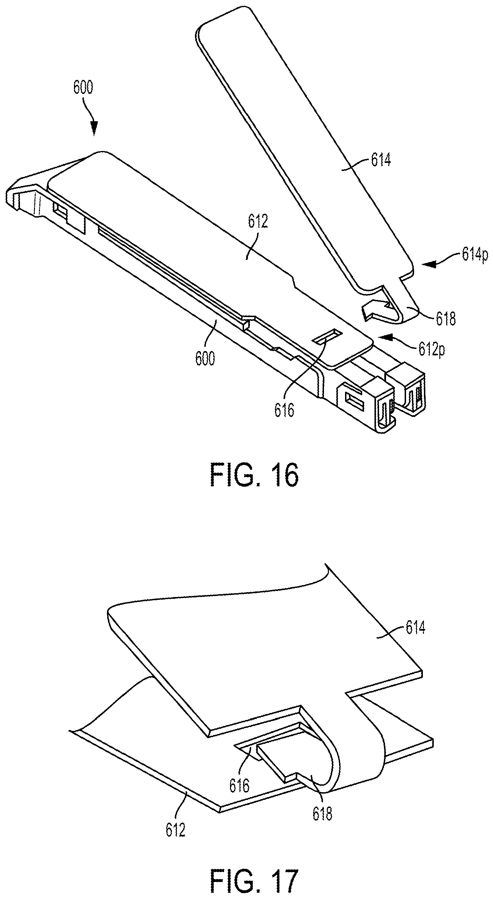

FIG. 16 illustrates another embodiment of an end effector 600, not forming part of the invention, having first and second adjunct materials 612, 614 releasably coupled thereto via respective mating features. FIG. 16 shows only a portion of the end effector 600, a cartridge body 602, which is configured to releasably retain thereon the first adjunct material 612. An opposed jaw, an anvil, which is not shown in FIG. 16, is configured to releasably retain thereon the second adjunct material 614.

In this implementation, the cartridge body 602 has the first adjunct material 612 releasably coupled thereto. The first adjunct material 612 has a size and shape complementary to a size and shape of a tissue-facing surface of the cartridge body 602 (obscured by the adjunct material 612), and the first adjunct material 612 can be coupled to the cartridge body 602 using adhesive material(s) or in other suitable ways.

In this embodiments, the first and second adjunct materials 612, 614 have respective mating features that are configured to couple (e.g., interlock) to one another to thereby couple the first and second adjunct materials 612, 614 to one another. Thus, the first adjunct material 612 has a slot 616 formed at a proximal end 612p thereof. The second adjunct material 614, which can have a size and shape complementary to a size and shape of a tissue-facing surface of the end effector's anvil (not shown), has a tab 618 extending from a proximal end 614p thereof and configured to be received within the slot 616. FIG. 17 shows the tab 618 of the second adjunct material 614 mating with the slot 616 of the first adjunct material 612. The tab 618 can have a length such that it engages with the slot 616 to retain the second adjunct material 614 in engagement with the first adjunct material 612. Also, in use, the tab 618, when engaged with the slot 616, can operate as a tissue stop to prevent or reduce tissue from being displaced or leaked from the treatment site when the jaws of the end effector 600 are grasping the tissue.

It should be appreciated that the slot 616 in the first adjunct material 612 and the tab 618 in the second adjunct material 614 are shown by way of example only, as the first and second adjunct materials 612, 614 can have any other mating features configured to couple with one another. In some embodiments, such mating features can be complementary to one another.

In some implementations, the first and second adjunct materials 612, 614 can be coupled to the respective jaws of the end effector 600 using other additional features. For example, adhesive can be used to releasably couple the adjunct materials 612, 614 to the jaws. Any other attachment features can be used additionally or alternatively.

In some embodiments, attachment features can be formed on an adjunct material configured to be releasably disposed on a jaw of an end effector. These embodiments can be used in connection with end effectors having gripping features that extend from a shorter side of each staple pocket in a cartridge of the end effector. For example, the gripping features can be implemented in accordance with ECHELON™ Gripping Surface Technology such that each of the staple pockets has opposed extension features configured to provide a grip that holds tissue in place during firing.

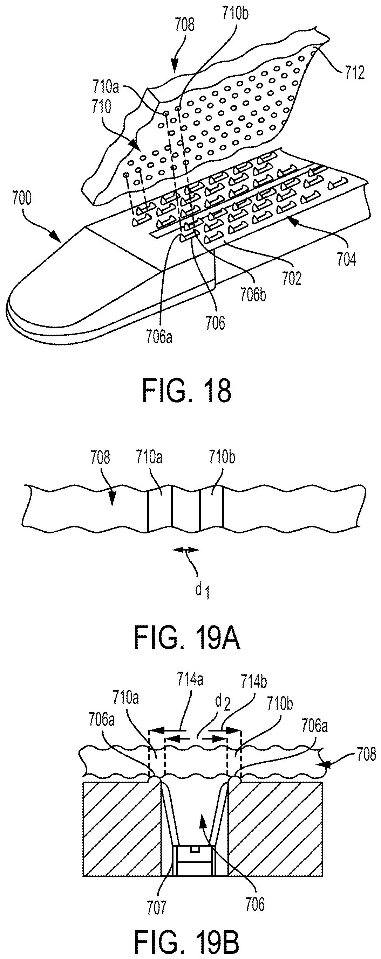

FIGS. 18, 19A and 19B illustrate an embodiment of a cartridge 700 of an end effector (not shown), not forming part of the invention, having gripping extensions formed at staple pockets 704 in a tissue-contacting surface 702. As shown in FIGS. 18 and 19B, each staple pocket has extension features formed at opposite sides of the pocket along a longitudinal axis of the pocket. For example, a staple pocket 706 has first and second extension features 706a, 706b formed at opposed sides of the pocket 706 along a longitudinal axis thereof. Some or all of the other staple pockets can be configured in a similar manner. The extension features 706a, 706b of the staple pocket 706 typically are formed integrally with the tissue-contacting surface 702.

The extension features 706a, 706b of the staple pocket 706 (as well as extension features of other staple pockets) can have any of a variety of configurations. For example, as shown in FIG. 19B, illustrating by way of example the staple pocket 706 in cross-section, the extension features 706a, 706b can be in the form of slightly curved features that are also slightly inclined towards a mid-portion of the staple pocket 706. It should be appreciated, however, that the extension features 706a, 706b can have any other configurations and that the described techniques are not limited to any particular type of extension features adjacent to staple pockets.

In the illustrated example, an adjunct material 708 is configured to be releasably retained on the tissue-contacting surface 702 of the cartridge 700. The adjunct material 708 can have a plurality of mating features for releasably mating with the cartridge 700. In particular, in this implementation, the mating features are in the form of openings 710 formed on the side 712 of the adjunct material 708 facing the tissue-contacting surface 702. The openings 710 are configured to mate with the extension features formed on the cartridge 700. For example, openings 710a, 710b shown in FIG. 18 are configured to mate with the extension features 706a, 706b, respectively, such that the extension features 706a, 706b are received within the openings 710a, 710b. Thus, the openings 710 can be configured to fit the extension features 706a, 706b therewithin, such that the size of the openings 710 corresponds to the size of the extension features 706a, 706b. Also, the openings 710 can be spaced from one another in accordance with a distance of the extension features 706a, 706b from one another, as discussed in more detail below.

The openings 710 can be formed on the adjunct 708 at predetermined locations such that each pair of openings (e.g., the openings 710a, 710b) is configured to be mated with corresponding extension features (e.g., the extension features 706a, 706b). The adjunct 708 can have openings formed thereon that correspond to each of the staple pocket's extension features, or, in some embodiments, only some of the openings can mate with the extension features, and vice versa. In other words, the number of the openings may be different from the number of extension features.

The openings in the adjunct can be relatively small. For example, in at least one embodiment, they can have a diameter of about 0.254 mm (0.010 inches), though the openings can have another diameter. In some implementations, the openings can be formed in the adjunct at certain distances from one another without taking into considerations specific pairs of extension features to mate with the pairs of openings. In such implementations, for example, when the adjunct is disposed over a cartridge and some force is applied thereto (e.g., the adjunct is pressed over a tissue-contacting surface of the cartridge), all or at least some of the openings will "find" extensions features to mate with, and vice versa. Thus, the adjunct can have multiple openings at a certain distance from one another (which can be smaller than a distance between the openings that are configured to mate with specific extension features) and at least some of these openings can mate with the extension features of the cartridge.

As mentioned above, in the example illustrated, the openings 710 can be spaced from one another in accordance with a distance of the extension features 706a, 706b from one another. Furthermore, as in the example illustrated, the adjunct material 708 can be made from at least partially stretchable material such that, when it is placed over the cartridge 700 (which can be done with application of some force), one or more portions of the adjunct material 708 can stretch. For example, the portions between at last some of the openings 710 can stretch so that the openings at opposed sides of the portions are placed in positions for mating with respective extension features.

Thus, as illustrated in FIGS. 19A and 19B, before the adjunct 708 is placed on the cartridge 700, the openings 710a, 710b (as also shown in FIG. 18) are disposed at a distance d1 from one another. When the adjunct 708 is caused into engagement with the cartridge 700 when force is applied thereto (via a user's hand or using any removable applicator, frame, etc.), the adjunct 708 is caused to stretch such that the openings 710a, 710b move further apart from one another, as shown by arrows 714a, 714b in FIG. 19B. In particular, the openings 710a, 710b become spaced apart at a distance d2 that is greater than the distance d1 (FIG. 19A), as shown in FIG. 19B. In this way, the openings 710a, 710b become disposed at the distance from one another that allows them to mate with the extension features 706a, 706b of the staple pocket 706 having a staple 707 ejectably disposed therein. When the adjunct 708 is caused to engage the tissue-contacting surface 702 of the cartridge 700, portion(s) of the adjunct 708, including the portion between the openings 710a, 710b can be displaced until one or both of the openings receives the corresponding extension feature therein. In other words, the adjunct 708 can be stretched, until the openings 710a, 710b in the adjunct 708 engage with the corresponding extension features 706a, 706b. Other openings in the adjunct engage the extension features in the cartridge in a similar manner, and the adjunct 708 thus becomes releasably engaged with the cartridge 700. In embodiments in which the number of openings in the adjunct is greater that the number of extension features, at least some of the openings can engage with the extension features in a similar manner.

A person skilled in the art will appreciate that the present invention has application in conventional minimally-invasive and open surgical instrumentation as well application in robotic-assisted surgery.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/ replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended
claims.

## Claims

1. An end effector (102) for a surgical instrument, comprising:
a first jaw having a cartridge (104) with a plurality of staple cavities configured to seat staples therein, the staple cavities opening on a tissue-facing surface (106) of the cartridge (104);
a second jaw opposing the first jaw and having an anvil with a plurality of staple forming cavities formed on a tissue-facing surface thereof, wherein the first and second jaws are configured to clamp tissue therebetween;
an attachment feature comprising a retaining filament (108) having
an intermediate portion (110);
first and second ends (112a, 112b) disposed on opposed sides of the intermediate portion (110), each of the first and second ends (112a, 112b) having an end feature configured to mate with at least one jaw of the first and second jaws; and
an adjunct material (100) releasably retained on the at least one jaw by the attachment feature that is arranged such that at least a part of the intermediate portion (110) is disposed over the adjunct material (100) and such that the first and second ends (112a, 112b) are spaced apart,
the end effector further comprising at least one first retaining member (118a) disposed at one side of a tissue-facing surface (106) of the at least one jaw in proximity to one edge of the tissue-facing surface (106) of the at least one jaw, and at least one second retaining member (118b) disposed at another, opposed side of the tissue-facing surface (106) of the at least one jaw in proximity to another, opposed edge of the tissue-facing surface (106), the first and second retaining members (118a, 118b) being configured to engage the part of the intermediate portion (110) disposed over the adjunct material (100),
**characterized in that** at least one of the first and second retaining members (118a, 118b) comprises a pair of adjacent posts (122d, 122p), each configured to engage at least a part of the intermediate portion (110) of the retaining filament (108).

2. The end effector of claim 1, wherein the adjunct material comprises at least one cut-out (126a, 126b) formed therein, the adjunct material being releasably retained on the at least one jaw such that at least one of the first and second retaining members is disposed within the cut-out.

3. The end effector of claim 1 or claim 2, wherein the first and second end features are disposed on a side of the at least one jaw that is opposed to a tissue-facing surface thereof, such that the first and second ends are spaced apart across a cutting element channel (113) of the at least one jaw extending longitudinally across a mid-portion of the jaw.

4. The end effector of any one of the preceding claims, wherein at least the intermediate portion of the retaining filament is coupled to the at least one jaw using an adhesive material.

5. The end effector of any one of the preceding claims, further comprising at least one roughened portion (130a, 130b) on a side of the at least one jaw that is opposed to a tissue-facing surface thereof, wherein the intermediate portion of the retaining filament encompasses opposed sides walls of the at least one jaw, and wherein the first and second ends comprise leaf members (114a, 114b) configured to frictionally engage a corresponding roughened portion of the at least one roughened portion.

6. The end effector of claim 5, wherein the leaf members are formed by heat pressing opposed end portions of the retaining filament.

7. The end effector of claim 5 or claim 6, further comprising at least one second roughened portion (210) on at least one of the opposed side walls, wherein portions of the intermediate portion of the retaining filament that encompass the opposed side walls frictionally engage a corresponding second roughened portion on one of the side walls.

8. The end effector of any one of the preceding claims, further comprising at least one spindle-type retaining member (306a, 306b) formed on a side of the at least one jaw that is opposed to a tissue-facing surface thereof, and wherein at least one of the first and second ends is configured to be retained via the spindle-type retaining member.

9. The end effector of claim 8, wherein the at least one spindle-type retaining member has at least one recess (308a, 308b, 310a, 310b) formed thereon and configured to retain therein a portion of the at least one of the first and second ends.

10. The end effector of any one of the preceding claims, further comprising
a first pair of recesses (414a, 414b) formed in a tissue-facing surface of the at least one jaw, the first pair of recesses being spaced from opposed edges of the tissue-facing surface, and
a second pair of recesses (418a, 418b) formed in the adjunct material at locations thereof corresponding to the first pair of recesses,
wherein parts of the intermediate portion of the retaining filament are configured to extend through the second pair of recesses so that the first and second ends are allowed within the first pair of recesses.

11. The end effector of claim 10, wherein the first and second ends comprise deformable elements (412a, 412b) configured to be reversibly deformed when allowed within the first pair of recesses.

12. The end effector of claim 11, wherein the deformable elements comprise t-shaped barb members.

13. The end effector of any one of claims 10 to 12, wherein the at least one jaw is the first jaw, and wherein the first pair of recesses is formed at a distal end of the jaw outside an outer perimeter of the plurality of staple cavities.

## Patentansprüche

1. Endeffektor (102) für ein chirurgisches Instrument, umfassend:
eine erste Backe, die ein Magazin (104) mit einer Vielzahl von Klammerhohlräumen, die zum Aufnehmen von Klammern darin gestaltet sind, aufweist, wobei die Klammerhohlräume an einer gewebezugewandten Oberfläche (106) des Magazins (104) offen sind;
eine zweite Backe gegenüber der ersten Backe, die ein Gegenlager mit einer Vielzahl von klammerformenden Hohlräumen an einer gewebezugewandten Oberfläche davon gebildet aufweist, wobei die erste und die zweite Backe dafür gestaltet sind, Gewebe dazwischen zu klemmen;
ein Befestigungselement, das einen Haltefaden (108) umfasst, der aufweist:
einen mittleren Teil (110);
ein erstes und ein zweites Ende (112a, 112b), die an gegenüberliegenden Seiten des mittleren Teils (110) angeordnet sind, wobei jedes von dem ersten und dem zweiten Ende (112a, 112b) ein Endelement aufweist, das dafür gestaltet ist, mit wenigstens einer Backe von der ersten und der zweiten Backe zusammenzupassen; und
ein Hilfsmaterial (100), das durch das Befestigungselement, das so angeordnet ist, dass wenigstens ein Teil des mittleren Teils (110) über dem Hilfsmaterial (100) angeordnet ist und das erste und das zweite Ende (112a, 112b) voneinander beabstandet sind, lösbar an der wenigstens einen Backe festgehalten wird,
wobei der Endeffektor ferner wenigstens ein erstes Halteelement (118a) umfasst, das an einer Seite einer gewebezugewandten Oberfläche (106) der wenigstens einen Backe nahe einem Rand der gewebezugewandten Oberfläche (106) der wenigstens einen Backe angeordnet ist, und wenigstens ein zweites Halteelement (118b), das an einer anderen, gegenüberliegenden Seite der gewebezugewandten Oberfläche (106) der wenigstens einen Backe nahe einem anderen, gegenüberliegenden Rand der gewebezugewandten Oberfläche (106) angeordnet ist, wobei das erste und das zweite Halteelement (118a, 118b) für den Eingriff mit dem Teil des mittleren Teils (110), der über dem Hilfsmaterial (110) angeordnet ist, gestaltet sind,
**dadurch gekennzeichnet, dass** wenigstens eines von dem ersten und dem zweiten Halteelement (118a, 118b) ein Paar von benachbarten Stiften (122d, 122p) umfasst, die jeweils für den Eingriff mit wenigstens einem Teil des mittleren Teils (110) des Haltefadens (108) gestaltet sind.

2. Endeffektor gemäß Anspruch 1, wobei das Hilfsmaterial wenigstens einen darin gebildeten Ausschnitt (126a, 126b) umfasst, wobei das Hilfsmaterial lösbar an der wenigstens einen Backe gehalten wird, so dass wenigstens eines von dem ersten und dem zweiten Halteelement innerhalb des Ausschnitts angeordnet ist.

3. Endeffektor gemäß Anspruch 1 oder Anspruch 2, wobei das erste und das zweite Endelement an einer Seite der wenigstens einen Backe angeordnet sind, die einer gewebezugewandten Seite davon gegenüberliegt, so dass das erste und das zweite Element über einen Schneidelement-Kanal (113) der wenigstens einen Backe, der sich längslaufend über einen mittleren Abschnitt der Backe erstreckt, beabstandet sind.

4. Endeffektor gemäß einem der vorstehenden Ansprüche, wobei wenigstens der mittlere Teil des Haltefadens unter Verwendung eines Haftmaterials an die wenigstens eine Backe gekoppelt ist.

5. Endeffektor gemäß einem der vorstehenden Ansprüche, ferner umfassend wenigstens einen aufgerauten Teil (130a, 130b) an einer Seite der wenigstens einen Backe, der einer gewebezugewandten Seite davon gegenüberliegt, wobei der mittlere Teil des Haltefadens gegenüberliegende Seitenwände der wenigstens einen Backe umgibt und wobei das erste und das zweite Ende Blattelemente (114a, 114b) umfassen, die zum Reibungseingriff eines entsprechenden aufgerauten Teil des wenigstens einen aufgerauten Teils gestaltet sind.

6. Endeffektor gemäß Anspruch 5, wobei die Blattelemente durch Warmpressen von gegenüberliegenden Teilen des Haltefadens gebildet sind.

7. Endeffektor gemäß Anspruch 5 oder Anspruch 6, ferner umfassend wenigstens einen zweiten aufgerauten Teil (210) an wenigstens einer der gegenüberliegenden Seitenwände, wobei Teile des mittleren Teils des Haltefadens, das die gegenüberliegenden Seitenwände umgibt, in Reibungseingriff mit einem entsprechenden zweiten aufgerauten Teil an einer der Seitenwände steht.

8. Endeffektor gemäß einem der vorstehenden Ansprüche, ferner umfassend wenigstens ein Halteelement (306a, 306b) vom Spindeltyp, das an einer Seite der wenigstens einen Backe gebildet ist, die einer gewebezugewandten Oberfläche davon gegenüberliegt, und wobei wenigstens eines von dem ersten und dem zweiten Ende dafür gestaltet ist, über das Halteelement vom Spindeltyp gehalten zu werden.

9. Endeffektor gemäß Anspruch 8, wobei das wenigstens eine Halteelement vom Spindeltyp wenigstens eine Aussparung (308a, 308b, 310a, 310b) darin gebildet aufweist, dafür gestaltet, einen Teil des wenigstens einen von dem ersten und dem zweiten Ende darin festzuhalten.

10. Endeffektor gemäß einem der vorstehenden Ansprüche, ferner umfassend
ein erstes Paar von Aussparungen (414a, 414b), die in einer gewebezugewandten Oberfläche der wenigstens einen Backe gebildet sind, wobei das erste Paar von Aussparungen von gegenüberliegenden Rändern der gewebezugewandten Oberfläche beabstandet ist, und
ein zweites Paar von Aussparungen (418a, 418b), die in dem Hilfsmaterial an Stellen davon gebildet sind, die dem ersten Paar von Aussparungen entsprechen,
wobei Teile des mittleren Teils des Haltefadens dafür gestaltet sind, durch das zweite Paar von Aussparungen zu verlaufen, so dass das erste Ende und das zweite Ende in dem ersten Paar von Aussparungen aufgenommen werden.

11. Endeffektor gemäß Anspruch 10, wobei das erste und das zweite Ende verformbare Elemente (412a, 412b) umfassen, die dafür gestaltet sind, reversibel verformt zu werden, wenn sie in dem ersten Paar von Aussparungen aufgenommen werden.

12. Endeffektor gemäß Anspruch 11, wobei die verformbaren Elemente T-förmige Hakenelemente umfassen.

13. Endeffektor gemäß einem der Ansprüche 10 bis 12, wobei die wenigstens eine Backe die erste Backe ist und wobei das erste Paar von Aussparungen an einem distalen Ende der Backe außerhalb des Außenumfangs der Vielzahl von Klammerhohlräumen gebildet ist.

## Revendications

1. Effecteur terminal (102) pour un instrument chirurgical, comprenant :
une première mâchoire comportant une cartouche (104) dotée d'une pluralité de cavités pour agrafes conçues pour asseoir des agrafes en leur sein, les cavités pour agrafes donnant sur une surface tournée vers un tissu (106) de la cartouche (104) ;
une seconde mâchoire en regard de la première mâchoire et comportant une enclume dotée d'une pluralité de cavités de formation d'agrafes formées sur une surface tournée vers un tissu de celle-ci, dans lequel les première et seconde mâchoires sont conçues pour pincer un tissu entre celles-ci ;
une caractéristique de fixation comprenant un filament de retenue (108) possédant
une partie intermédiaire (110) ;
des première et seconde extrémités (112a, 112b) disposées sur des côtés opposés de la partie intermédiaire (110), chacune des première et seconde extrémités (112a, 112b) possédant une caractéristique d'extrémité conçue pour s'apparier avec au moins une mâchoire des première et seconde mâchoires ; et
un matériau auxiliaire (100) retenu de manière libérable sur l'au moins une mâchoire par la caractéristique de fixation qui est agencée de sorte qu'au moins une partie de la partie intermédiaire (110) soit disposée sur le matériau auxiliaire (100) et de sorte que les première et seconde extrémités (112a, 112b) soient espacées,
l'effecteur terminal comprenant en outre au moins un premier élément de retenue (118a) disposé au niveau d'un côté d'une surface tournée vers un tissu (106) de l'au moins une mâchoire à proximité d'un bord de la surface tournée vers un tissu (106) de l'au moins une mâchoire, et au moins un second élément de retenue (118b) disposé au niveau d'un autre côté opposé de la surface tournée vers un tissu (106) de l'au moins une mâchoire à proximité d'un autre bord opposé de la surface tournée vers un tissu (106), les premier et
second éléments de retenue (118a, 118b) étant conçus pour entrer en prise avec la partie de la partie intermédiaire (110) disposée sur le matériau auxiliaire (100),
**caractérisé en ce qu'**au moins un des premier et second éléments de retenue (118a, 118b) comprend une paire de montants adjacents (122d, 122p), conçus chacun pour entrer en prise avec au moins une partie de la partie intermédiaire (110) du filament de retenue (108).

2. Effecteur terminal selon la revendication 1, dans lequel le matériau auxiliaire comprend au moins une découpe (126a, 126b) formée en son sein, le matériau auxiliaire étant retenu de manière libérable sur l'au moins une mâchoire de sorte qu'au moins un des premier et second éléments de retenue soit disposé à l'intérieur de la découpe.

3. Effecteur terminal selon la revendication 1 ou revendication 2, dans lequel les première et seconde caractéristiques d'extrémité sont disposées sur un côté de l'au moins une mâchoire qui est en regard d'une surface tournée vers un tissu de celle-ci, de sorte que les première et seconde extrémités soient espacées sur toute l'étendue d'un canal (113) d'élément de coupe de l'au moins une mâchoire s'étendant longitudinalement en travers d'une partie médiane de la mâchoire.

4. Effecteur terminal selon l'une quelconque des revendications précédentes, dans lequel au moins la partie intermédiaire du filament de retenue est accouplée à l'au moins une mâchoire à l'aide d'un matériau adhésif.

5. Effecteur terminal selon l'une quelconque des revendications précédentes, comprenant en outre au moins une partie rugosifiée (130a, 130b) sur un côté de l'au moins une mâchoire qui est en regard d'une surface tournée vers un tissu de celle-ci, dans lequel la partie intermédiaire du filament de retenue englobe des parois latérales opposées de l'au moins une mâchoire, et dans lequel les première et seconde extrémités comprennent des éléments en forme de feuille (114a, 114b) conçus pour entrer en prise par frottement avec une partie rugosifiée correspondante de l'au moins une partie rugosifiée.

6. Effecteur terminal selon la revendication 5, dans lequel les éléments en forme de feuille sont formés par le thermopressage de parties terminales opposées du filament de retenue.

7. Effecteur terminal selon la revendication 5 ou revendication 6, comprenant en outre au moins une seconde partie rugosifiée (210) sur au moins une des parois latérales opposées, dans lequel des parties de la partie intermédiaire du filament de retenue qui englobent les parois latérales opposées entrent en prise par frottement avec une seconde partie rugosifiée correspondante sur une des parois latérales.

8. Effecteur terminal selon l'une quelconque des revendications précédentes, comprenant en outre au moins un élément de retenue de type tige (306a, 306b) formé sur un côté de l'au moins une mâchoire qui est en regard d'une surface tournée vers un tissu de celle-ci, et dans lequel au moins une parmi les première et seconde extrémités est conçue pour être retenue par le biais de l'élément de retenue de type tige.

9. Effecteur terminal selon la revendication 8, dans lequel l'au moins un élément de retenue de type tige possède au moins un renfoncement (308a, 308b, 310a, 310b) formé sur celui-ci et conçu pour retenir en son sein une partie de l'au moins une des première et seconde extrémités.

10. Effecteur terminal selon l'une quelconque des revendications précédentes, comprenant en outre
une première paire de renfoncements (414a, 414b) formée dans une surface tournée vers un tissu de l'au moins une mâchoire, la première paire de renfoncements étant espacée de bords opposés de la surface tournée vers un tissu, et
une seconde paire de renfoncements (418a, 418b) formée dans le matériau auxiliaire au niveau d'emplacements de celui-ci correspondant à la première paire de renfoncements,
dans lequel des parties de la partie intermédiaire du filament de retenue sont conçues pour s'étendre à travers la seconde paire de renfoncements de sorte que les première et seconde extrémités soient autorisées à l'intérieur de la première paire de renfoncements.

11. Effecteur terminal selon la revendication 10, dans lequel les première et seconde extrémités comprennent des éléments déformables (412a, 412b) conçus pour être déformés de manière réversible lorsqu'ils sont autorisés à l'intérieur de la première paire de renfoncements.

12. Effecteur terminal selon la revendication 11, dans lequel les éléments déformables comprennent des éléments de barbe en t.

13. Effecteur terminal selon l'une quelconque des revendications 10 à 12, dans lequel l'au moins une mâchoire est la première mâchoire, et dans lequel la première paire de renfoncements est formée au niveau d'une extrémité distale de la mâchoire à l'extérieur d'un périmètre externe de la pluralité de cavités pour agrafes.
